# EUROPÄISCHE PATENTANMELDUNG

(11) **EP 4 566 701 A1**
(43) Veröffentlichungstag der Anmeldung: **11.06.2025**
(21) Anmeldenummer: 23214789.2
(22) Anmeldetag: 07.12.2023
(51) Int. Cl.: B01F 23/50, B01F 31/40, B01F 33/501, B01F 101/20

(54) **KIT ZUM HERSTELLEN UND AUSPRESSEN VON KNOCHENZEMENT UND VERFAHREN**

(71) Anmelder: Heraeus Medical GmbH, 61273 Wehrheim (DE)
(72) Erfinder: Vogt, Sebastian, 61273 Wehrheim (DE); Kluge, Thomas, 61273 Wehrheim (DE); Hohmann, Ekaterini, 61273 Wehrheim (DE)
(74) Vertreter: Heraeus IP

(57) **Zusammenfassung**

Ein Kit zum Herstellen und Auspressen von Knochenzement, eine Verwendung eines Kits und ein Verfahren. Ein Kit (1) zum Herstellen und Auspressen von Knochenzement (15), umfasst einen ersten Behälter (12), der eine erste Komponente (81) zur Herstellung von Knochenzement (15) enthält, einen zweiten Behälter (70), der eine zweite Komponente (82) zur Herstellung von Knochenzement (15) enthält, eine Mischvorrichtung (74) zum manuellen Mischen der ersten Komponente (81) und der zweiten Komponente (82) im ersten Behälter (12) zwecks Herstellung von Knochenzement (15) und eine Auspressvorrichtung (10) zum manuellen Auspressen des hergestellten Knochenzements (15) aus dem ersten Behälter (12). Auf diese Weise werden alle für das Herstellen und Auspressen des Knochenzements benötigten Stoffe und Vorrichtungen gemeinsam bereitgestellt.

## Beschreibung

Die Erfindung betrifft einen Kit zum Herstellen und Auspressen von Knochenzement, eine Verwendung eines Kits sowie ein Verfahren zur Verwendung eines Kits.

Knochenzement wird üblicherweise durch Mischen eines Pulvers mit einer Flüssigkeit hergestellt. Beispielsweise sind Polymethylmethacrylat-(PMMA)-Knochenzemente bekannt, die aus einer flüssigen Monomerkomponente und einer Pulverkomponente zusammengesetzt sind. Die Monomerkomponente enthält im Allgemeinen das Monomer Methylmethacrylat und insbesondere einen darin gelösten Aktivator wie z. B. N,N-Dimethyl-p-toluidin. Die Pulverkomponente, auch als Knochenzementpulver bezeichnet, weist ein oder mehrere Polymere, die auf Basis von Methylmethacrylat und Comonomeren, wie Styren, Methylacrylat oder ähnlichen Monomeren durch Polymerisation, vorzugsweise Suspensionspolymerisation, hergestellt sind und insbesondere einen Röntgenopaker und/oder den Initiator Dibenzoylperoxid auf. Beim Vermischen der Pulverkomponente mit der Monomerkomponente entsteht, z. B. durch Quellung der Polymere der Pulverkomponente im Methylmethacrylat, ein plastisch verformbarer Teig, der eigentliche Knochenzement (auch als Knochenzementteig bezeichnet). Beim Vermischen der Pulverkomponente mit der Monomerkomponente reagiert beispielsweise der Aktivator N,N-Dimethyl-p-toluidin mit Dibenzoylperoxid unter Bildung von Radikalen. Die gebildeten Radikale können die radikalische Polymerisation des Methylmethacrylates initiieren. Mit fortschreitender Polymerisation des Methylmethacrylates kann sich die Viskosität des Zements erhöhen, bis dieser erstarrt.

PMMA-Knochenzemente werden hauptsächlich zur dauerhaften Fixierung von Gelenkendoprothesen im Knochen verwendet. Dabei werden im allgemeinen Zementmengen von 50 g bis 125 g oder mehr zur Fixierung einer Gelenkendoprothese verwendet. Der Knochenzement wird dabei während der Operation des Patienten benötigt und sollte entsprechend zügig bereitgestellt werden. Dies ist das wesentliche Anwendungsgebiet der vorliegenden Erfindung. Das Anwendungsgebiet der Erfindung liegt dagegen nicht in der Bereitstellung von Kleinstmengen im Bereich von weniger als 10 g bzw. 10 ml Knochenzement zur Stabilisierung von Wirbelkörpern, wie dies z. B. bei der Kyphoplastie und der Vertebroplastie der Fall ist. Derartige Kleinstmengen können mit einfachen technischen Mitteln ausgepresst werden.

Polymethylmethacrylat-Knochenzemente können in geeigneten Mischbechern mit Hilfe von Spateln durch Vermischen des Zementpulvers mit der Monomerflüssigkeit vermischt werden. Dabei kann es zum Einschluss von Luftblasen im Knochenzement kommen, welche die mechanischen Eigenschaften des ausgehärteten Knochenzements negativ beeinflussen können. Andere Nachteile sind die Notwendigkeit, die Mengen manuell abzumessen und die nicht immer sichergestellte ausreichende Durchmischung. Die hier genannten Merkmale, Ausführungsformen und Definitionen können beliebig mit den Merkmalen der Erfindung kombiniert werden.

Zur Vermeidung von Lufteinschlüssen im Knochenzement sind Vakuum-Zementiersysteme bekannt, beispielsweise US 6 033 105 A, US 5 624 184 A, US 4 671 263 A, US 4 973 168 A, US 5 100 241 A, WO 99/67015 A1, EP 1 020 167 A2, US 5 586 821 A, EP 1 016 452 A2, DE 36 40 279 A1, WO 94/26403 A1, EP 1 005 901 A2, EP 1 886 647 A1 und US 5 344 232 A. Hier besteht die Notwendigkeit, ein Vakuum anzulegen. Eine Weiterentwicklung in der Zementiertechnik stellen Zementiersysteme dar, in denen sowohl das Zementpulver als auch die Monomerflüssigkeit bereits in separaten Kompartimenten verpackt sind und unmittelbar vor der Zementapplikation im Zementiersystem miteinander vermischt werden. Solche geschlossenen Full-Prepacked-Mischsysteme oder ähnliche Systeme sind unter anderen in den Dokumenten EP 0 380 867 B1, EP 0 796 653 B1, EP 0 692 229 A1, DE 10 2009 031 178 B3, US 5 997 544 A, US 6 709 149 B1, WO 00/35506 A1 und EP 0 796 653 A2 beschrieben. Es ist bislang nötig, eine externe Vorrichtung zum Herauspressen, beispielsweise eine Kartuschenpresse (Zementpistole), zu verwenden. Da derartige Vorrichtungen teuer und kompliziert sind, werden diese separat bereitgestellt und können dann in Verbindung mit dem entsprechenden Mischsystem verwendet werden. Anschließend ist eine Reinigung und Sterilisation nötig, um die Vorrichtung erneut benutzen zu können.

Mischvorrichtungen, bei denen eine externe Vorrichtung zum Herauspressen des Knochenzements verwendet wird, oder ähnliche Systeme sind in WO9416951A1, EP1741413B1, EP3054880B1 und DE19718648A1 offenbart. Mischvorrichtungen, bei denen ein Vakuum angelegt wird, um den Transport oder das Mischen einer Komponente oder des Knochenzements zu ermöglichen, oder ähnliche Systeme sind aus US8662736B2 und EP3093067B1 bekannt. EP2393456B1 beschreibt eine Mischvorrichtung, bei der die Flüssigkeit mittels eines Überdrucks in ein Pulver gedrückt wird.

Manuell zu bedienende Auspressvorrichtungen basieren beispielsweise auf manuell bewegbaren Hebelsystemen, die Schubstangen oder Zahnstangen antreiben und die mit den Zementkartuschen verbunden werden. Durch wiederholte Kippbewegung der Hebel wird die Schubstange oder Zahnstange in Richtung des Austragskolbens der Kartuschen bewegt, wobei der Austragskolben den Polymethylmethacrylat-Knochenzement durch Bewegung in Richtung Kartuschenkopf aus der Kartusche mit davor angebrachten Austragsrohren herauspresst. Derartige oder ähnliche Auspressvorrichtungen sind in US589344A, US5638997A und WO9416951A offenbart.

Für den Austrag von kleinen Zementmengen bis zu ungefähr 10 g Knochenzement sind Schraubsysteme üblich, die Verwendung für die Stabilisierung von frakturierten Wirbelkörpern finden. Diese bisher bekannten Schraubsysteme sind mechanisch nur gering belastbar und nicht für den Austrag größerer Mengen von mindestens 50 g PMMA-Knochenzement geeignet. Exemplarisch für solche Austragsvorrichtungen oder ähnliche Vorrichtungen sind die Publikationen US6676663B2, US2012224452A1, CN218220290U und CN213190021U.

Es ist die Aufgabe der Erfindung, eine einfache und kostengünstige Lösung zum Herstellen und Auspressen von Knochenzement bereitzustellen.

Die Aufgabe wird gelöst durch einen Kit zum Herstellen und Auspressen von Knochenzement gemäß Anspruch 1 sowie eine Verwendung eines Kits und ein Verfahren gemäß den nebengeordneten Ansprüchen.

Zur Lösung der Aufgabe dient ein Kit zum Herstellen und Auspressen von Knochenzement, umfassend:
- einen ersten Behälter, der eine erste Komponente zur Herstellung von Knochenzement enthält,
- einen zweiten Behälter, der eine zweite Komponente zur Herstellung von Knochenzement enthält,
- eine Mischvorrichtung zum manuellen Mischen der ersten Komponente und der zweiten Komponente im ersten Behälter zwecks Herstellung von Knochenzement, sowie
- eine Auspressvorrichtung zum manuellen Auspressen des hergestellten Knochenzements aus dem ersten Behälter.

Der Kit enthält typischerweise alle für das Herstellen und Auspressen benötigten Stoffe und Vorrichtungen und kann daher auch als Full-Prepacked-Mischsystem oder als "Procedure Pack" bezeichnet werden. Zudem enthält der Kit eine Auspressvorrichtung. Deshalb ist das Kit dazu geeignet, Knochenzement nicht nur herzustellen, sondern auch am Einsatzort auszupressen. Da die herkömmlichen Auspressvorrichtungen teuer und technisch aufwändig sind, werden diese grundsätzlich nicht in einen Kit integriert.

Der Kit kann vom medizinischen Anwender, z. B. dem operierenden Arzt, während der Operation direkt verwendet werden. In einer Ausführungsform ist der Kit so ausgestaltet, dass keine weiteren Vorrichtungen oder Gegenstände notwendig sind, um den Knochenzement herzustellen und auszupressen. In einer anderen Ausführungsform ist der Kit so ausgestaltet, dass er mit einer Vakuumquelle verbunden werden kann, sodass zum Mischen des Knochenzements ein Vakuum an den ersten Behälter angelegt werden kann.

Insbesondere kann Knochenzement durch Mischen der ersten Komponente und der zweiten Komponente hergestellt werden. Es ist jedoch nicht ausgeschlossen, dass für die Herstellung des Knochenzements ein oder mehrere zusätzliche Stoffe vorhanden sind. Insbesondere umfasst oder ist die erste Komponente ein Pulver, beispielsweise umfassend eines oder mehrere Polymere. Insbesondere umfasst oder ist die zweite Komponente eine Flüssigkeit, beispielsweise eine Monomerflüssigkeit.

Der erste Behälter umschließt einen Hohlraum, in dem die erste Komponente enthalten ist und in den die zweite Komponente eingeführt werden kann. Der erste Behälter kann darüber hinaus weitere Bestandteile umfassen wie einen oder mehrere Verbindungsbereiche wie unten beschrieben.

Insbesondere ist der erste Behälter ein geschlossener Behälter. Insbesondere ist der erste Behälter als Kartusche ausgestaltet. Die Kartusche umfasst insbesondere eine zylinderförmige, beispielsweise kreiszylinderförmige, Wandung. Die Auspressvorrichtung kann so ausgestaltet sein, dass ein Stempel von einer ersten Seite entlang der Längsrichtung der Kartusche in die Kartusche eingepresst werden kann, um den darin befindlichen Knochenzement zu verdrängen. Auf diese Weise kann der Knochenzement an der gegenüberliegenden zweiten Seite ausgepresst werden.

Insbesondere ist der zweite Behälter ein geschlossener Behälter. Beispielsweise kann der zweite Behälter als Beutel oder Ampulle ausgebildet sein. Es können zwei oder mehr zweite Behälter vorhanden sein.

Insbesondere ist die Auspressvorrichtung manuell bedienbar. Damit ist gemeint, dass eine manuelle Kraft insbesondere von außen auf den ersten Behälter ausgeübt werden kann, die auf mechanischem Wege ein Auspressen des Knochenzements ermöglicht. Insbesondere ist die Auspressvorrichtung dazu eingerichtet, mechanisch mit dem ersten Behälter verbunden zu werden. So kann die Auspressvorrichtung beispielsweise erst dann mit dem ersten Behälter verbunden werden, wenn das Mischen des Knochenzements im ersten Behälter fertiggestellt ist. Die Verbindung zwischen der Auspressvorrichtung und dem ersten Behälter kann reversibel, nämlich manuell lösbar sein. Bevorzugt umfasst die Auspressvorrichtung einen Verbindungsbereich und der erste Behälter umfasst einen Verbindungsbereich, wobei die beiden Verbindungsbereiche manuell und insbesondere reversibel miteinander verbunden werden können. Im verbundenen Zustand kann Knochenzement mit der Auspressvorrichtung aus dem ersten Behälter ausgepresst werden. die Verbindungsbereiche können beispielsweise als Gewinde, als Überwurfbügel oder bevorzugt als Bajonettverschluss ausgestaltet sein. Beide Verbindungsbereiche umfassen dann entsprechende Verbindungselemente, z. B. in Form von Zapfen. Die Verbindungselemente des ersten Behälters können im Inneren einer Wandung des ersten Behälters angeordnet sein und/oder nach innen ragen. Damit ist ein Auspressen von Knochenzement wenige Sekunden nach der Beendigung des Mischens möglich. Es kann dadurch wertvolle Operationszeit eingespart werden. Zudem kann der Benutzer schneller beginnen zu zementieren, so dass bis zum Ende der Verarbeitungsphase ein längerer Zeitraum zum Zementieren bleibt.

Die Mischvorrichtung ist insbesondere dazu eingerichtet, ein Mischelement im ersten Behälter zu bewegen, um die beiden Komponenten zu vermischen. Beispielsweise umfasst die Mischvorrichtung einen Handgriff, mit dem das Mischelement im ersten Behälter bewegt werden kann. Handgriff und Mischelement sind typischerweise über einen zentralen bzw. axialen Stab miteinander verbunden. Die Bewegung erfolgt beispielsweise auf und ab entlang der Längsachse des ersten Behälters und/oder drehend um die Längsachse. Das Mischelement kann eine durchbrochene Scheibe sein, die beim Bewegen Teile der Masse im ersten Behälter verdrängt und andere Teile der Masse durchlässt, um eine Durchmischung zu erreichen. Die Außenkontur der Scheibe kann im Wesentlichen oder vollständig der Innenkontur des ersten Behälters entsprechen, so dass die Scheibe wie ein Zylinder in einem Kolben bewegt werden kann.

Bevorzugt umfasst die Mischvorrichtung einen Verbindungsbereich und der erste Behälter umfasst einen Verbindungsbereich, wobei die beiden Verbindungsbereiche manuell und insbesondere reversibel miteinander verbunden werden können. Im verbundenen Zustand können die Komponenten im ersten Behälter durch die Mischvorrichtung miteinander gemischt werden, um Knochenzement herzustellen.

In einer Ausführungsform umfasst der Kit ferner mindestens ein Austragsrohr zum Austragen des hergestellten Knochenzements, einen Pressurizer wie beispielsweise einen Kniepressurizer und/oder einen Hüftpressurizer, einen Schnorchel wie beispielsweise einen Knieschnorchel und/oder einen Flachschnorchel, und/oder einen Vakuumschlauch zur Verbindung des ersten Behälters mit einer externen Vakuumquelle. Ein Pressurizer ist ein aus flexiblem Material wie Gummi hergestelltes Verbindungsstück, mit dem Knochenzement aus dem ersten Behälter in Körpergewebe wie Spongiosa gedrückt werden kann. Ein Schnorchel ist ein Schlauchkörper zum Transportieren von Knochenzement an eine Zielposition insbesondere im Bereich eines Gelenks.

In einer Ausgestaltung ist die Mischvorrichtung zumindest teilweise im ersten Behälter angeordnet. Mit anderen Worten befindet sich zumindest ein Teil der Mischvorrichtung, insbesondere das Mischelement, im ersten Behälter. Dies ermöglicht eine besonders platzsparende Verpackung und reduziert die Anzahl der Arbeitsschritte bei der Verwendung. Die Mischvorrichtung kann so weit wie möglich in den ersten Behälter eingeschoben sein. Typischerweise befindet sich ein Handgriff der Mischvorrichtung außerhalb des ersten Behälters. Der Handgriff kann das Einschieben begrenzen.

Die Mischvorrichtung kann fertig montiert, also einsatzbereit, vorliegen. Alternativ ist nur ein Teil der Mischvorrichtung, insbesondere die Adaptereinheit, mit dem ersten Behälter verbunden, während ein anderer Teil der Mischvorrichtung, insbesondere die Gewindestange mit dem Handgriff, separat angeordnet ist. In diesem Fall muss die Gewindestange vor dem Mischen mit der Adaptereinheit verbunden werden.

Axial im Sinne der Erfindung bezieht sich auf eine jeweilige Längsachse. Die axiale Erstreckung ist die Länge zwischen den gegenüberliegenden Enden der jeweiligen Komponente. Die axiale Erstreckung der Auspressvorrichtung bezieht sich demnach auf die Länge der Auspressvorrichtung entlang ihrer Längsachse. Typischerweise ist die Auspressvorrichtung dazu eingerichtet, entlang der Längsachse bewegt zu werden und/oder Knochenzement entlang der Längsachse zu bewegen. Die axiale Erstreckung des ersten Behälters mit der Mischvorrichtung bezieht sich auf die Länge der Gruppe aus diesen beiden Bestandteilen im beschriebenen Zustand, in dem die Mischvorrichtung zumindest teilweise im ersten Behälter angeordnet ist.

In einer Ausgestaltung weist die Auspressvorrichtung eine axiale Erstreckung A auf und der erste Behälter mit der Mischvorrichtung weist eine axiale Erstreckung B auf. Für das Verhältnis A / B kann gelten: A / B ≥ 0,9, insbesondere ≥ 1,0 oder ≥ 1,1 und/oder A / B ≤ 1,5, insbesondere ≤ 1,4 oder ≤ 1,3.

Mit anderen Worten ist die Auspressvorrichtung höchstens um den Faktor 1,5 größer als der erste Behälter mit der Mischvorrichtung. Eine kleine Auspressvorrichtung ermöglicht so einen kompakten Kit mit geringem Packmaß. Beispielsweise ist die Auspressvorrichtung höchstens geringfügig kleiner, typischerweise aber zumindest genau so groß wie der erste Behälter mit der Mischvorrichtung. Dies stellt sicher, dass der Knochenzement zumindest weitgehend und insbesondere vollständig aus dem ersten Behälter ausgepresst werden kann und dennoch keiner der Bestandteile übermäßig lang im Vergleich zu den anderen Bestandteilen ist. Die Auspressvorrichtung ist aber insbesondere nicht größer als das 1,4-fache des ersten Behälters. So kann ein besonders platzsparendes Packmaß erreicht werden. Zudem kann eine besonders kompakte Form erreicht werden, bei der die Länge und die Breite des Kits in ähnlichen Bereichen liegen. Beispielsweise ist die Länge höchstens 50%, bevorzugt höchstens 35% und insbesondere höchstens 20% größer als die Breite. Es hat sich gezeigt, dass derartige Abmessungen vorteilhaft hinsichtlich Transport und Aufstellung während der Verwendung aufweisen. Zudem ist die Standsicherheit erhöht.

Nach vollständigem Auspressen des Knochenzements befindet sich die Auspressvorrichtung typischerweise teilweise in dem ersten Behälter. Die axiale Erstreckung des ersten Behälters mit der Auspressvorrichtung ist C. In einer Ausführungsform gilt für das Verhältnis: C / A ≤ 1,4.

In einer Ausführungsform ist die maximale Höhe des Kits, insbesondere eines Packmittels des Kits, geringer als die Länge und/oder die Breite. Insbesondere beträgt die Höhe maximal 70% der Länge und/oder der Breite, bevorzugt maximal 55% der Länge und/oder der Breite. Auf diese Weise wird eine hohe Standsicherheit des Kits gewährleistet.

In einer Ausführungsform ist nach dem vollständigen Auspressen des Knochenzements die axiale Erstreckung des ersten Behälters mit der darin befindlichen Auspressvorrichtung, insbesondere vom äußersten Punkt des ersten Behälters bis zum Ende des Handgriffs der Auspressvorrichtung auf der gegenüberliegenden Seite, gleich oder kleiner der 1,2-fachen axialen Erstreckung der Auspressvorrichtung.

Die Auspressvorrichtung kann ein Hebelsystem aufweisen und/oder als Kartuschenpresse ausgestaltet sein. Die Auspressvorrichtung kann durch eine Gaspatrone angetrieben sein, beispielsweise wie in EP 2 711 091 B1 und/oder EP 2 710 972 B1 beschrieben. Jedes dieser Dokumente wird durch Bezugnahme in diese Anmeldung einbezogen.

In einer Ausgestaltung weist die Auspressvorrichtung eine Gewindestange mit einem Außengewinde, einen Handgriff zum Drehen der Gewindestange sowie eine Adaptereinheit zum mechanischen Verbinden mit dem ersten Behälter auf. Insbesondere weist die Adaptereinheit eine Durchgangsöffnung mit einem Innengewinde für die Gewindestange auf. Der erste Behälter ist dann entsprechend dazu eingerichtet, mit der Adaptereinheit mechanisch verbunden zu werden.

Insbesondere ist ein Verbindungsmittel der Auspressvorrichtung zur Verbindung mit dem ersten Behälter an der Adaptereinheit vorgesehen. Insbesondere ist dazu eine drehfeste Verbindung zumindest in der Drehrichtung der Gewindestange vorgesehen, sodass das Drehen der Gewindestange die Befestigung der Adaptereinheit an dem ersten Behälter nicht beeinträchtigt oder löst. Ist die Adaptereinheit am ersten Behälter befestigt, bewirkt die axiale Relativbewegung der Gewindestange in Bezug zur Adaptereinheit und damit zum ersten Behälter, dass das dem Handgriff gegenüberliegenden Ende der Gewindestange in den ersten Behälter eindringen kann und so Knochenzement aus dem ersten Behälter herauspressen kann. Auf diese Weise kann aus dem der Adaptereinheit gegenüberliegenden Ende des ersten Behälters Knochenzement herausgepresst werden.

In einer Ausführungsform beträgt eine Steigung des Außengewindes wenigstens 1 mm, bevorzugt wenigstens 3 mm und/oder höchstens 7 mm, bevorzugt höchstens 5 mm. Dies hat sich in Versuchen als besonders zielführend erwiesen. In einer Ausführungsform beträgt ein Außendurchmesser des Außengewindes der Gewindestange wenigstens 12 mm und/oder höchstens 17 mm. Der Außendurchmesser meint den maximalen Durchmesser, der zwischen den zwei maximalen Erhebungen des Gewindegangs gemessen wird, also den Durchmesser eines das Außengewinde umgebenden, gedachten Kreiszylinders. Insbesondere beträgt der Außendurchmesser wenigstens 13,5 mm und/oder höchstens 15 mm. In einer Ausführungsform beträgt der Außendurchmesser etwa oder genau 14 mm. Ein Außendurchmesser unterhalb von 12 mm ist nicht geeignet, den auftretenden Kräften bzw. Momenten in geeigneter Weise standzuhalten. Ein zu großer Außendurchmesser erhöht die Reibung und damit die für ein Austragen benötigte Kraft. In einer Ausführungsform enthält das Innengewinde der Adaptereinheit wenigstens 2 Gewindegänge, bevorzugt wenigstens oder genau 4 Gewindegänge und/oder höchstens 6 Gewindegänge, bevorzugt höchstens 5 Gewindegänge. In einer Ausführungsform ist das Gewinde der Gewindestange ein Trapezgewinde. Dies hat sich als besonders geeignet erwiesen. In einer Ausführungsform weist das Außengewinde der Gewindestange eine Gewindetiefe von wenigstens 1 mm und/oder höchstens 3 mm oder 4 mm auf.

In einer Ausführungsform weist die Auspressvorrichtung an dem Ende der Gewindestange, welches dem Handgriff gegenüberliegt, ein Druckelement zum Ausüben eines Drucks auf den in dem ersten Behälter befindlichen Knochenzement auf. Insbesondere hat das Druckelement eine flache Oberfläche. Das Druckelement kann zum direkten oder indirekten Ausüben des Drucks eingerichtet sein. In einer Ausführungsform ist das Druckelement drehfest mit der Gewindestange verbunden. Beispielsweise kann das Druckelement mit der Gewindestange verschraubt, verschweißt oder mittels einer Steckverbindung mit der Gewindestange verbunden sein. Das Druckelement kann ganz oder teilweise aus Metall oder Kunststoff, beispielsweise aus glasfaserverstärktem Kunststoff und/oder Polyamid, hergestellt sein. In einer Ausführungsform ist das Druckelement zweiteilig aufgebaut. Ein erster Teil des Druckelements ist drehfest mit der Gewindestange verbunden. Ein zweiter Teil des Druckelements ist um die Längsachse der Gewindestange drehbar mit dem ersten Teil verbunden. Es kann zwischen dem ersten Teil des Druckelements ein Schmiermittel wie z. B. Silikon vorgesehen sein, um eine besonders leichtgängige relative Drehung zwischen dem ersten Teil und dem zweiten Teil zu gewährleisten. In einer Ausführungsform umgreift der zweite Teil den ersten Teil zumindest abschnittsweise oder umlaufend. Mit anderen Worten bildet der zweite Teil eine Hinterschneidung hinter dem ersten Teil. Auf diese Weise wird gewährleistet, dass der erste Teil und der zweite Teil sich nicht voneinander lösen. Eine axiale Bewegung des zweiten Teils weg vom ersten Teil und damit ein Verlieren des zweiten Teils wird so verhindert. Insbesondere ist eine dem ersten Teil zugewandte Oberfläche des zweiten Teils und/oder eine dem zweiten Teil zugewandte Oberfläche des ersten Teils flach und/oder glatt ausgeführt. Das Druckelement und/oder jedes der Teile ist insbesondere beständig gegen eine Druckkraft von wenigstens 1 kN, bevorzugt von 2 kN.

Diese Merkmale ermöglichen einen besonders einfachen manuellen Austrag größerer Mengen an Knochenzement, wie sie für die Fixierung einer Gelenkendoprothese im menschlichen Knochengewebe benötigt werden. Die Auspressvorrichtung kann vollständig oder teilweise kostengünstig durch Kunststoffspritzguss hergestellt werden.

In einer Ausgestaltung beträgt die äußere Oberfläche eines Gewindegangs des Außengewindes höchstens 370 mm². In einer Ausgestaltung beträgt die äußere Oberfläche eines Gewindegangs des Außengewindes wenigstens 230 mm².

Die äußere Oberfläche hat einen starken Einfluss auf die Reibungsverluste beim Drehen der Gewindestange in der Adaptereinheit und damit auf die für das Austragen des Knochenzements aufzubringende Kraft. Die äußere Oberfläche ist ein Maß für die Fläche des Außengewindes, die das Innengewinde kontaktiert. Deshalb ist die entstehende Reibung proportional zu der äußeren Oberfläche. Versuche haben gezeigt, dass es dabei unerheblich ist, ob eine Verringerung der äußeren Oberfläche durch eine Reduktion der Gewindetiefe, eine Erhöhung des Flankenwinkels, eine Reduktion des Durchmessers der Gewindestange, eine Reduktion der Steigung oder anderen oder durch Kombinationen von unterschiedlichen Maßnahmen erzielt wird. Maßgeblich ist lediglich der absolute Wert der äußeren Oberfläche.

Durch die erfindungsgemäße Reduzierung der Reibung können auch Personen mit geringerer Kraft die Auspressvorrichtung ohne Schwierigkeiten bedienen. Dies ist insbesondere bedeutsam, da sich der Knochenzement mit fortschreitender Dauer nach dem Anmischen weiter verfestigt und daher im Laufe der Zeit die für das Auspressen benötigten Kräfte noch steigen. Versuche haben gezeigt, dass bei der beschriebenen maximalen äußeren Oberfläche eines Gewindegangs des Außengewindes für jeden Benutzer ein einfaches Austragen des Knochenzements durch manuelles Drehen des Handgriffs möglich ist. Bei größeren äußeren Oberflächen bewirken die Haft- und Gleitreibung ein sehr schwergängiges Austragen und damit eine schlechte Handhabbarkeit. Diese Ausgestaltung erlaubt es, 125 g Polymethylmethacrylat-Knochenzement, der typischerweise hochviskos ist, innerhalb weniger Sekunden auszupressen.

Die untere Grenze der äußeren Oberfläche bewirkt, dass die mechanische Stabilität der Gewindestange gewährleistet bleibt. Bei zu geringen äußeren Oberflächen sind beispielsweise die Gewindetiefe oder der Durchmesser der Gewindestange so gering, dass es zu einem Abscheren des Außengewindes und/oder zu einer Torsion bzw. einem Knicken der Gewindestange kommen kann.

Auch die Steigung des Außengewindes beeinflusst die für das Austragen benötigte Kraft. Die Steigung des Außengewindes ist der in axialer Richtung gemessene Weg, der bei einer Umdrehung zurückgelegt wird. Grundsätzlich führt eine zu geringe Steigung zu einer sehr hohen Übersetzung und damit zu einem erhöhten Aufwand bei der Benutzung, da eine größere Anzahl Drehungen zurückgelegt werden muss, um den Knochenzement auszutragen. Dies kann dazu führen, dass eine zu lange Zeit benötigt wird, was insbesondere während einer Operation und/oder bei einer fortschreitenden Verhärtung des Knochenzements unerwünscht ist. Grundsätzlich führt eine zu hohe Steigung zu einer geringen Übersetzung und damit zu einer sehr hohen Kraft, um den Knochenzement auszutragen. Zudem kann bei zu hohen Steigungen die Selbsthemmung verringert werden oder wegfallen, was wiederum die Bedienung unnötig verkompliziert. Die konkreten Zahlenwerte dieser Effekte sind von unterschiedlichen Rahmenbedingungen wie z. B. den verwendeten Materialien und Eigenschaften der jeweiligen Oberflächen abhängig.

Die Auspressvorrichtung kann aus Kunststoff oder Kunststoff mit Metall hergestellt sein. Eine komplizierte Mechanik wie im Falle einer Kartuschenpresse ist nicht notwendig. Es werden keine Hebel, Bolzen, Stifte, Klemmungen, Rastelemente, Zahnstangen und/oder Zahnräder benötigt. Die für ein Herauspressen des Knochenzements notwendigen Kräfte sind geringer als die Kräfte, die mit herkömmlichen Auspressvorrichtungen benötigt werden.

Die erfindungsgemäße Auspressvorrichtung ist einfach zu handhaben, da keine komplizierte Bedienung einer Kartuschenpresse notwendig ist. Das Austragen erfolgt einfach durch Drehen des Handgriffs.

Ein Gewindegang meint eine Schraubenlinie des Gewindes über eine Strecke, die einer vollständigen relativen Drehung um 360° entspricht. Es wird also eine Umdrehung betrachtet. Die äußere Oberfläche ist die Fläche des Außengewindes, die das korrespondierende Innengewinde kontaktieren kann. Die äußere Oberfläche eines Gewindegangs setzt sich beispielsweise zusammen aus der Fläche einer ersten (beispielsweise aufsteigenden) Gewindeflanke, einer zweiten (beispielsweise absteigenden) Gewindeflanke, einer Fläche im Bereich der Erhebung des Gewindes, die sich beispielsweise als parallel zur Längsachse erstrecken kann und/oder sich zwischen den Gewindeflanken befinden kann, und einer weiteren Fläche im Bereich der Vertiefung bzw. des Tals des Gewindes, die sich zwischen zwei Gewindeflanken befinden kann. Diese vier Flächen werden über eine Umdrehung addiert. Es wird mit anderen Worten die Fläche zwischen zwei benachbarten Erhebungen oder Tälern des Gewindes betrachtet. Zwischen den einzelnen Flächen befindliche Rundungen oder Übergänge sind ebenfalls Teil der äußeren Oberfläche. Jeweilige Kennwerte für das Außengewinde der Gewindestange können entsprechend für das Innengewinde der Adaptereinheit gelten.

Im Fall eines Trapezgewindes entspricht die äußere Oberfläche beispielsweise den Flächen der beiden Gewindeflanken, der dazwischen liegenden, nach außen weisenden Fläche im Bereich der Erhebung des Gewindes, die den Außendurchmesser des Außengewindes definiert, sowie der nach außen weisenden Fläche im Bereich des Tals des Gewindes, die den Innendurchmesser des Außengewindes definiert.

Im Falle eines mehrgängigen Gewindes werden die Flächen aller Gänge des Gewindes über eine Umdrehung addiert.

In einer Ausgestaltung ist die Auspressvorrichtung dazu eingerichtet, den Knochenzement mit einer Auspresskraft von mindestens 0,8 kN, insbesondere mindestens 1,0 kN, aus dem ersten Behälter zu pressen. Die Auspresskraft ist die Kraft, die auf den im ersten Behälter befindlichen Knochenzement wirkt. Im Falle einer aus Metall hergestellten Kartuschenpresse wirkt beispielsweise eine Auspresskraft von 2,5 kN. Im Falle geringer Mengen an Knochenzement für Rückenmarksanwendungen wirkt dagegen eine deutlich geringere Kraft. Bevorzugt ist die Auspressvorrichtung so ausgestaltet, dass eine Zementmenge zwischen 50 g und 125 g innerhalb einer Minute auspressbar ist.

In einer Ausgestaltung umfasst der Kit ein Packmittel. Die Bestandteile des Kits sind von dem Packmittel eingeschlossen, und zwar insbesondere luftdicht und/oder steril. Die Bestandteile sind der erste Behälter, der zweite Behälter, die Mischvorrichtung, die Auspressvorrichtung und ggf. weitere Bestandteile, wie beschrieben. Das Packmittel ist insbesondere aus Kunststoff hergestellt. Das Packmittel kann als Blister ausgebildet sein. Ein Blister umfasst eine beispielsweise tiefgezogene Schale, die einen Innenraum zur Anordnung der Bestandteile ausbildet, sowie einen Deckel, der insbesondere als Folie ausgestaltet ist. Typischerweise ist der Deckel planar, erstreckt sich also in einer Ebene. Die Schale und/oder der Deckel des Blisters kann, muss aber nicht notwendigerweise transparent sein.

In einer Ausgestaltung enthält der erste Behälter als erste Komponente eine Menge von mindestens 40 g eines Pulvers, beispielsweise eines PMMA-Knochenzementpulvers. In einer Ausgestaltung enthält der zweite Behälter als zweite Komponente eine Menge von mindestens 18 ml einer Flüssigkeit, beispielsweise einer Monomerflüssigkeit.

In einer Ausführungsform enthält der erste Behälter eine Menge von mindestens 20 g, bevorzugt mindestens 30 g und/oder höchstens 200 g, bevorzugt höchstens 120 g, insbesondere höchstens 86 g, der ersten Komponente. In einer Ausführungsform enthält der zweite Behälter eine Menge von mindestens 10 ml, bevorzugt mindestens 15 ml und/oder höchstens 100 ml, bevorzugt höchstens 75 ml, insbesondere höchstens 50 ml oder höchstens 42 ml der zweiten Komponente. Diese Mengen haben sich für die Fixierung von Gelenkendoprothesen im Knochen als optimal erwiesen.

In einer Ausgestaltung ist der zweite Behälter in einer Vorrichtung zum Lagern und Öffnen des zweiten Behälters angeordnet. Die Vorrichtung zum Lagern und Öffnen des zweiten Behälters ist fluidtechnisch mit dem ersten Behälter verbunden oder verbindbar, um die zweite Komponente in den ersten Behälter zu überführen.

Die Vorrichtung zum Lagern und Öffnen ist zum einen dazu eingerichtet, den mindestens einen zweiten Behälter zu lagern. Insbesondere umschließt die Vorrichtung den mindestens einen zweiten Behälter vollständig und bietet so einen mechanischen Schutz. Zudem umfasst die Vorrichtung ein Mittel zum Öffnen des Behälters. Im Falle eines Beutels kann dies beispielsweise eine Nadel bzw. ein Dorn sein. Im Falle einer Ampulle kann dies ein Brechmechanismus sein, der einen Teil der Ampulle, beispielsweise einen Kopf der Ampulle, von einem anderen Teil der Ampulle trennt bzw. die Ampulle aufbricht, beispielsweise durch Knicken oder Scheren. Es können zwei Beutel, beispielsweise beidseitig des ersten Behälters, und ggf. zwei Nadeln vorhanden sein. Um eine Nadel herum befindet sich typischerweise eine Abdichtung, die an der Wandung des Beutels anliegt.

Die Vorrichtung zum Lagern und Öffnen des zweiten Behälters kann eine Pumpe umfassen, insbesondere zur manuellen Betätigung, um die Flüssigkeit in den ersten Behälter zu pumpen. Beispielsweise ist die Vorrichtung gemäß EP 3 093 067 B1 ausgestaltet. Dieses Dokument wird durch Bezugnahme in diese Anmeldung einbezogen. Alternativ oder ergänzend kann die Vorrichtung zum Lagern und Öffnen des zweiten Behälters dazu eingerichtet sein, die Flüssigkeit schwerkraftbedingt in den ersten Behälter zu fördern. Auf diese Weise ist eine Verwendung des Kits unabhängig von einer externen Vakuumquelle möglich. Alternativ kann die Vorrichtung dazu eingerichtet sein, die Flüssigkeit mittels eines Vakuums in den ersten Behälter zu fördern. Beispielsweise ist die Vorrichtung gemäß EP 2 404 864 B1 oder EP 4 282 518 A1 ausgestaltet. Diese Dokumente werden durch Bezugnahme in diese Anmeldung einbezogen.

Diese Ausgestaltung ermöglicht ein Öffnen des zweiten Behälters zum Überführen der zweiten Komponente in den ersten Behälter. Die Verbindung zwischen der Vorrichtung und dem ersten Behälter ist bevorzugt reversibel, nämlich manuell lösbar. So kann die Vorrichtung zum Lagern und Öffnen nach erfolgter Überführung der zweiten Komponente vom ersten Behälter gelöst werden, um den Austrag des Knochenzements zu ermöglichen oder zu vereinfachen. Bevorzugt umfasst die Vorrichtung zum Lagern und Öffnen einen Verbindungsbereich und der erste Behälter umfasst einen Verbindungsbereich, wobei die beiden Verbindungsbereiche manuell und insbesondere reversibel miteinander verbunden werden können. Im verbundenen Zustand kann die zweite Komponente aus dem zweiten Behälter in den ersten Behälter überführt werden. Der Verbindungsbereich des ersten Behälters zur Verbindung der Vorrichtung zum Lagern und Öffnen kann der Verbindungsbereich des ersten Behälters sein, an dem auch die Mischeinrichtung angeschlossen werden kann, oder ein anderer Verbindungsbereich. Im zweiten Fall können die beiden Verbindungsbereiche des ersten Behälters an derselben Seite oder an gegenüberliegenden Seiten angeordnet sein.

In einer Ausgestaltung ist der erste Behälter und separat von der Auspressvorrichtung angeordnet. In einer Ausgestaltung ist der zweite Behälter separat von der Auspressvorrichtung angeordnet. Insbesondere ist die Vorrichtung zum Lagern und Öffnen des zweiten Behälters mitsamt dem zweiten Behälter separat von der Auspressvorrichtung angeordnet.

Mit anderen Worten ist der jeweilige Behälter räumlich getrennt von der Auspressvorrichtung angeordnet. Die jeweiligen Bestandteile können sich in demselben Raum eines Packmittels befinden, sind aber nicht unmittelbar mechanisch miteinander verbunden. Dies ermöglicht ein besonders geringes Packmaß der Bestandteile und damit eine geringe Größe des Kits. Die absolute Länge des Kits wird begrenzt, so dass Platz gespart werden kann. Die langen Bestandteile des Kits können parallel angeordnet sein. Insbesondere wird eine kompakte Form erreicht, bei der die Länge und die Breite des Kits in ähnlichen Bereichen liegen. Beispielsweise ist die Länge wie beschrieben nur in begrenzten Umfang größer als die Breite, so dass sich die beschriebenen Vorteile ergeben. Im mechanisch verbundenen Zustand des ersten Behälters mit der Auspressvorrichtung würde sich dagegen eine große Länge in Verbindung mit einer geringen Breite ergeben, sodass diese Vorteile nicht erreicht werden.

In einer Ausgestaltung ist die Mischvorrichtung zumindest teilweise im ersten Behälter angeordnet. Der erste Behälter mit der Mischvorrichtung und die Auspressvorrichtung sind parallel angeordnet, und zwar bevorzugt übereinander. Typischerweise ist die Auspressvorrichtung dabei unten angeordnet. Auf diese Weise kann eine besonders platzsparende Anordnung erreicht werden. Gleichzeitig wird eine Reihenfolge der Verwendung der Bestandteile während der Verwendung des Kits berücksichtigt. Dies ist der Fall, da der erste Behälter mit der Mischvorrichtung und die Auspressvorrichtung grundsätzlich nacheinander benötigt werden. Nach dem Herstellen des Knochenzements kann der Benutzer die Auspressvorrichtung entnehmen, mit dem ersten Behälter verbinden und den Knochenzement auspressen. Eine Fehlbedienung wird so verhindert, es ist kein zusätzlicher Platz zur zwischenzeitlichen Lagerung von Bestandteilen erforderlich und ein Herunterfallen von Bestandteilen wird vermieden. Die beschriebene Anordnung befindet sich insbesondere in einem Packmittel.

In einer Ausgestaltung bestehen der erste Behälter, der zweite Behälter, die Mischvorrichtung und die Auspressvorrichtung insgesamt zu maximal 20% aus Stahl oder zu maximal 20% aus Metall. Dies reduziert Kosten, Herstellungsaufwand und Gewicht des Kits. In einer Ausführungsform bestehen die genannten Bestandteile zu maximal 20% aus Stahl oder Metall oder enthalten keinen Stahl und/oder kein Metall. In einer Ausgestaltung besteht die Auspressvorrichtung maximal zu 50%, bevorzugt zu 30% aus Metall. Ist kein oder nur wenig Metall enthalten, kann der Kit bzw. die jeweiligen Bestandteile auf einfache Weise recycelt oder thermisch verwertet werden.

In einer Ausgestaltung ist die Adaptereinheit mit dem ersten Behälter mechanisch verbunden oder verbindbar. In einer Ausgestaltung weist die Adaptereinheit eine Aufstellfläche zum Aufstellen der Adaptereinheit mit dem ersten Behälter auf eine glatte Oberfläche und/oder auf eine Halterung des Packmittels auf. Die Adaptereinheit kann so als Aufstellmittel für den ersten Behälter dienen. Die Aufstellfläche der Adaptereinheit kann auf der Oberfläche oder der Halterung stehen. Die Adaptereinheit weist insbesondere an der der Aufstellfläche entgegengesetzten Seite, in axialer Richtung betrachtet, einen Verbindungsbereich zum Verbinden mit dem ersten Behälter auf. Die Adaptereinheit kann so auf ihrer Aufstellfläche stehen und gleichzeitig den ersten Behälter halten. Insbesondere weist das Packmittel eine Halterung auf, auf der die Aufstellfläche aufgestellt werden kann. Insbesondere korrespondieren Konturen der Halterung und der Aufstellfläche, und zwar bevorzugt dreidimensional, so dass ein besonders fester Halt erreicht werden kann.

In einer Ausführungsform umfasst die Vorrichtung zum Lagern und Öffnen des zweiten Behälters einen Knickbereich, an der Bereiche der Vorrichtung relativ zu einander bewegt, insbesondere gedreht, werden können. Der Knickbereich ist so eingerichtet, dass eine relative Bewegung zu einem Knicken führt, so dass der insbesondere als Ampulle ausgestaltete zweite Behälter zerbrochen wird. Insbesondere ist die Vorrichtung zumindest im Bereich des Knickbereichs flexibel ausgestaltet.

In einer Ausführungsform umfasst die Vorrichtung zum Lagern und Öffnen des zweiten Behälters eine Außenhülle, eine Innenhülle und ein Aufbrechelement. Allgemein dient ein Aufbrechelement zum Aufbrechen einer Ampulle. Insbesondere umgibt jede der zwei Hüllen einen Teil des als Ampulle ausgestalteten zweiten Behälters. Die Außenhülle und die Innenhülle sind relativ zu einander beweglich, insbesondere verschieblich. Bei einer relativen Bewegung der Außenhülle und der Innenhülle bewegt sich die Ampulle in Bezug zum Aufbrechelement. Das Aufbrechelement kann z. B. ausgestaltet sein wie in der Patentanmeldung EP23177510.7 beschrieben, die durch Bezugnahme in diese Anmeldung einbezogen wird. Das Aufbrechelement kann ein Scherelement sein. Das Scherelement ist so angeordnet, dass es bei einem relativen Verschieben der Außenhülle und der Innenhülle die Ampulle kontaktiert, insbesondere den Kopf der Ampulle, sodass die Ampulle geöffnet wird. Das Scherelement kann typischerweise eine Scherkraft auf den Kopf der Ampulle ausüben, so dass die Ampulle an der vorgesehenen Sollbruchstelle bricht. Diese Ausgestaltungen ermöglichen ein Herstellen des Knochenzements in einem geschlossenen System, bei dem ein Kontakt des Benutzers mit den Komponenten ausgeschlossen wird.

In einer Ausgestaltung umfasst der Kit zwei erste Behälter, die jeweils eine erste Komponente zur Herstellung von Knochenzement enthalten, zwei zweite Behälter, die jeweils eine zweite Komponente zur Herstellung von Knochenzement enthalten, und nur eine Auspressvorrichtung. Die Auspressvorrichtung kann wahlweise mit jedem der beiden ersten Behälter mechanisch verbunden werden, sodass hergestellter Knochenzement mit der Auspressvorrichtung nacheinander aus jedem der zwei ersten Behälter ausgepresst werden kann. Der Kit kann eine Mischvorrichtung aufweisen, die wahlweise mit jedem der beiden ersten Behälter mechanisch verbunden werden, um die jeweils zwei Komponenten zu mischen. Alternativ kann der Kit zwei Mischvorrichtungen aufweisen, um in jedem der zwei ersten Behälter mit einer separaten Mischvorrichtung mischen zu können.

In einer Ausführungsform sind der erste Behälter, der zweite Behälter, die Mischvorrichtung und/oder die Auspressvorrichtung wie in EP 2 281 532 B1, EP 2 269 718 B1, EP 3 093 067 B1 und/oder EP 22 174 728 A1 beschrieben ausgestaltet. Jedes dieser Dokumente wird durch Bezugnahme in diese Anmeldung einbezogen.

In einer Ausführungsform sind der erste Behälter, der zweite Behälter, die Mischvorrichtung und/oder die Auspressvorrichtung so eingerichtet, nach Öffnen des zweiten Behälters Monomerflüssigkeit mit Hilfe eines äußeren Vakuums in das Pulver gesaugt werden und anschließend beide Komponenten mit Hilfe der von außen zu bedienenden Mischvorrichtung vermischt werden können. Alternativ oder ergänzend kann die Überführung der zweiten Komponente in zwei Schritten erfolgen, wobei die Flüssigkeit zuerst durch Schwerkrafteinwirkung in ein Sammelvolumen fließen kann und anschließend aus diesem, beispielsweise durch einen manuell bedienbaren Pumpkolben, in den ersten Behälter gepumpt wird.

Ein weiterer Aspekt der Erfindung ist die Verwendung eines erfindungsgemäßen Kits zum Herauspressen von Knochenzement aus dem ersten Behälter. Insbesondere wird eine Menge von mindestens 50 g und/oder höchstens 130 g Knochenzement herausgepresst. Insbesondere erfolgt das Herauspressen mit Hilfe der Auspressvorrichtung. Insbesondere wird PMMA-Knochenzement ausgepresst. Insbesondere erfolgt dies zur Fixierung einer Gelenkendoprothese im menschlichen Knochengewebe. Insbesondere wird der Handgriff einer Auspressvorrichtung manuell gedreht, so dass Knochenzement aus den ersten Behälter herausgepresst wird. Alle Merkmale, Vorteile und Ausgestaltungen des oben genannten Kits und seiner Bestandteile und Eigenschaften gelten auch für die Verwendung sowie für das unten stehende Verfahren und umgekehrt.

Ein weiterer Aspekt der Erfindung ist ein Verfahren zur Verwendung eines erfindungsgemäßen Kits. Dieses umfasst:
- Mischen der ersten Komponente mit der zweiten Komponente im ersten Behälter unter Verwendung der Mischvorrichtung zur Herstellung von Knochenzement,
- Entnahme der Auspressvorrichtung aus einem Packmittel des Kits, sowie
- Auspressen des hergestellten Knochenzements aus dem ersten Behälter unter Verwendung der Auspressvorrichtung.

Das Verfahren kann ferner einen oder mehrere der folgenden Schritte in beliebiger Kombination umfassen:
- Öffnen eines Packmittels,
- Entnehmen des ersten Behälters und/oder des zweiten Behälters aus einem Packmittel,
- Öffnen des zweiten Behälters und Überführung der zweiten Komponente in den ersten Behälter,
- Verbinden der Mischvorrichtung mit dem ersten Behälter,
- Nach dem Mischen: Entfernen der Mischvorrichtung vom ersten Behälter,
- Verbinden der Auspressvorrichtung mit dem ersten Behälter,
- Verbinden eines Schlauchkörpers zum Auspressen mit dem ersten Behälter, insbesondere an einer der Auspressvorrichtung gegenüberliegenden Seite des ersten Behälters.

Nachfolgend werden Ausführungsbeispiele der Erfindung auch anhand von Figuren näher erläutert. Merkmale der Ausführungsbeispiele können einzeln oder in einer Mehrzahl mit den beanspruchten Gegenständen kombiniert werden, sofern nichts Gegenteiliges angegeben ist. Die beanspruchten Schutzbereiche sind nicht auf die Ausführungsbeispiele beschränkt.

Es zeigen:
- Figur 1:: einen erfindungsgemäßen Kit in Draufsicht,
- Figur 2:: einen erfindungsgemäßen Kit in Seitenansicht,
- Figur 3:: einen erfindungsgemäßen Kit in einer perspektivischen Ansicht,
- Figur 4:: einen erfindungsgemäßen Kit in Draufsicht,
- Figur 5:: Bestandteile eines erfindungsgemäßen Kits,
- Figur 6:: eine Komponente eines erfindungsgemäßen Kits,
- Figur 7:: Bestandteile eines erfindungsgemäßen Kits,
- Figur 8:: Bestandteile eines erfindungsgemäßen Kits,
- Figur 9:: Bestandteile eines erfindungsgemäßen Kits,
- Figur 10:: Bestandteile eines erfindungsgemäßen Kits,
- Figur 11:: Bestandteile eines erfindungsgemäßen Kits,
- Figur 12:: Bestandteile eines erfindungsgemäßen Kits,
- Figur 13:: eine Schnittdarstellung einer Auspressvorrichtung,
- Figur 14:: eine weitere Schnittdarstellung einer Auspressvorrichtung,
- Figur 15:: Details einer Auspressvorrichtung in einem ersten Behälter,
- Figur 16:: ein vergrößertes Detail einer Gewindestange, sowie
- Figur 17:: mehrere Ansichten eines ersten Behälters an bzw. in einem Packmittel.

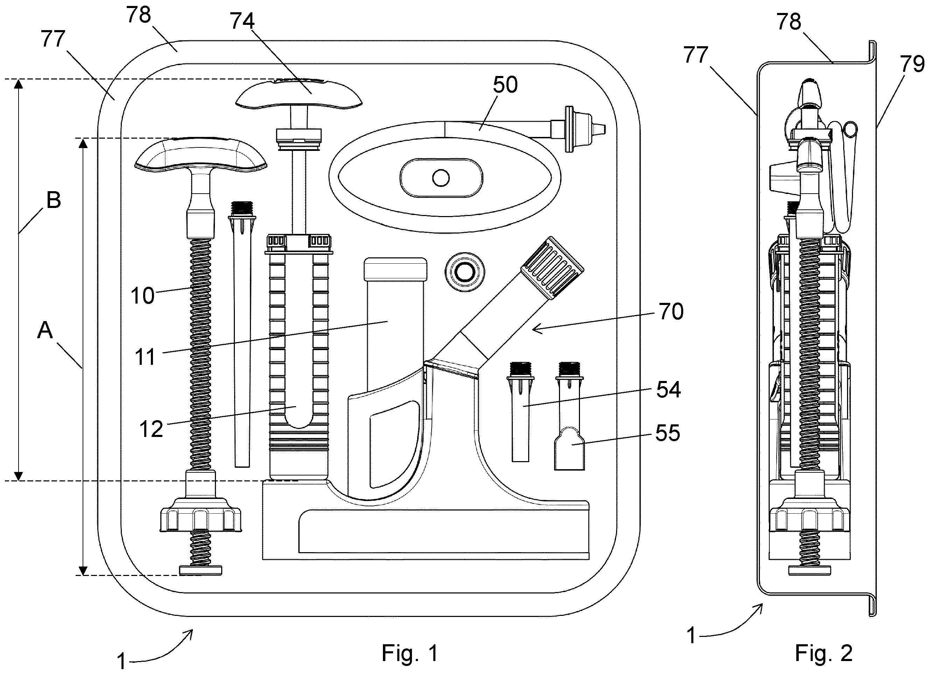

Die Figuren 1 und 2 zeigen einen erfindungsgemäßen Kit 10 zum Herstellen und Auspressen von Knochenzement. Der Kit umfasst einen ersten Behälter 12, einen zweiten Behälter 70, eine Auspressvorrichtung 10 und eine Mischvorrichtung 74. Die Mischvorrichtung 74 ist mechanisch mit dem ersten Behälter 12 verbunden, sodass nach dem Überführen der zweiten Komponente aus dem zweiten Behälter 70 in den ersten Behälter 12, der die erste Komponente enthält, die beiden Komponenten mit der Mischvorrichtung 74 gemischt werden können, um Knochenzement zu erhalten. Der Kit enthält zudem einen Knieschnorchel 54 und einen Flachschnorchel 55 sowie einen Vakuumschlauch 50 zum Verbinden des ersten Behälters 12 mit einer Vakuumquelle. Durch Vakuum kann vorteilhaft die zweite Komponente in den ersten Behälter 12 gesaugt werden. Allerdings ist mit der hier gezeigten Ausführungsform auch ein Betrieb ohne Vakuum möglich. Rechts neben dem ersten Behälter 12 ist eine Pumpeinrichtung 11 angeordnet, der zum Pumpen der Flüssigkeit in den ersten Behälter genutzt werden kann. Die Pumpeinrichtung kann so ausgestaltet werden wie in DE 10 2015 106 899 B3 beschrieben, was durch Bezugnahme in diese Anmeldung einbezogen wird.

Der Kit umfasst ferner ein Packmittel 77, in dem die Komponenten insbesondere eingepackt sind. Das Packmittel 77 umfasst eine insbesondere tiefgezogene Schale 78 mit einem verbreiterten, umlaufenden Rand sowie einen Deckel 79, der auf den Rand aufgebracht ist, beispielsweise verschweißt oder verklebt. Das Packmittel 77 hat eine im Wesentlichen quadratische Grundform.

Die Mischvorrichtung 74 ist teilweise im ersten Behälter 12 angeordnet. Für die axiale Erstreckung A der Auspressvorrichtung 10 und die axiale Erstreckung B des ersten Behälters 12 mit der Mischvorrichtung 74 gilt A/ B ist zwischen 1,0 und 1,2. Im hier gezeigten Fall beträgt A / B ungefähr 1,1.

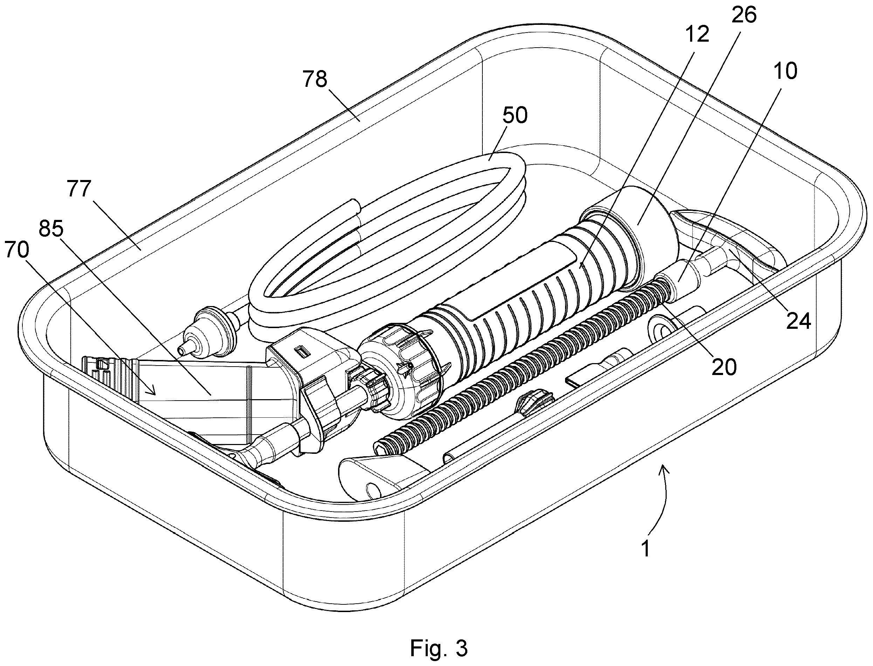

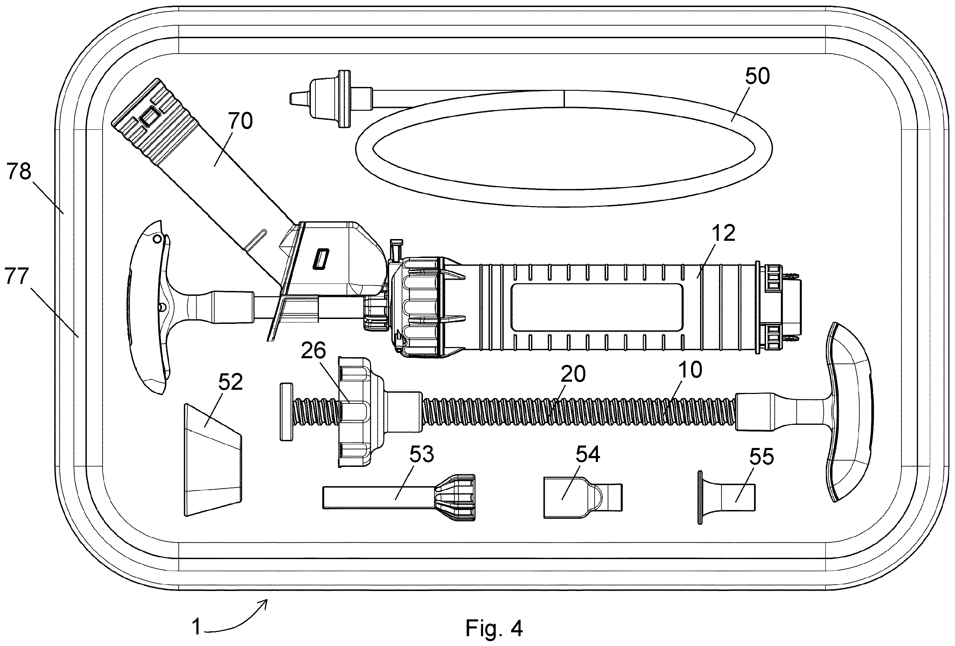

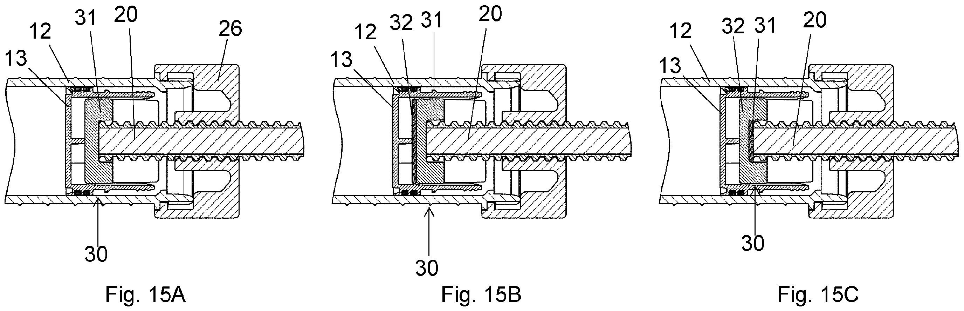

Die Figuren 3 und 4 zeigen andere Ausgestaltungen eines Kits 1, bei dem das Packmittel 77 eine längliche Grundform hat. In der hier gezeigten Ausführungsform ist im Kit 1 eine Vorrichtung 85 zum Lagern und Öffnen des zweiten Behälters enthalten, die den zweiten Behälter 70 enthält. Die Vorrichtung 85 ist mechanisch insbesondere reversibel mit dem ersten Behälter 12 verbunden. Dies erleichtert die Verwendung, da die Verbindung nicht erst hergestellt werden muss. In Figur 3 ist eine zweigeteilte Auspressvorrichtung 10 enthalten. Eine Adaptereinheit 26 der Auspressvorrichtung ist mechanisch mit einem Ende des ersten Behälters 12 verbunden, und zwar an der der Vorrichtung 85 gegenüberliegenden Seite. Ein Handteil der Auspressvorrichtung 10 mit Gewindestange 20 und Handgriff 24 ist separat davon und/oder daneben angeordnet. Die Adaptereinheit 26 kann an der dem ersten Behälter 12 abgewandten Seite in Bezug zur axialen Erstreckung eine Aufstellfläche zum Aufstellen auf eine glatte Oberfläche und/oder auf eine Halterung des Packmittels aufweisen (vergl. Fig. 15A bis 15C). Dies erleichtert das Aufstellen des ersten Behälters 12 zum Mischen und/oder nach dem Mischen.

In der in Figur 4 gezeigten Ausführungsform ist dagegen die Auspressvorrichtung 10 vollständig separat von und/oder neben dem ersten Behälter 12 angeordnet. Die Gewindestange 20 ist hierbei in die Adaptereinheit 26 eingeschraubt. Der Kit 1 umfasst ferner einen Vakuumschlauch 50, einen Kniepressurizer 52, einen Hüftpressurizer 53, einen Knieschnorchel 54 und einen Flachschnorchel 55.

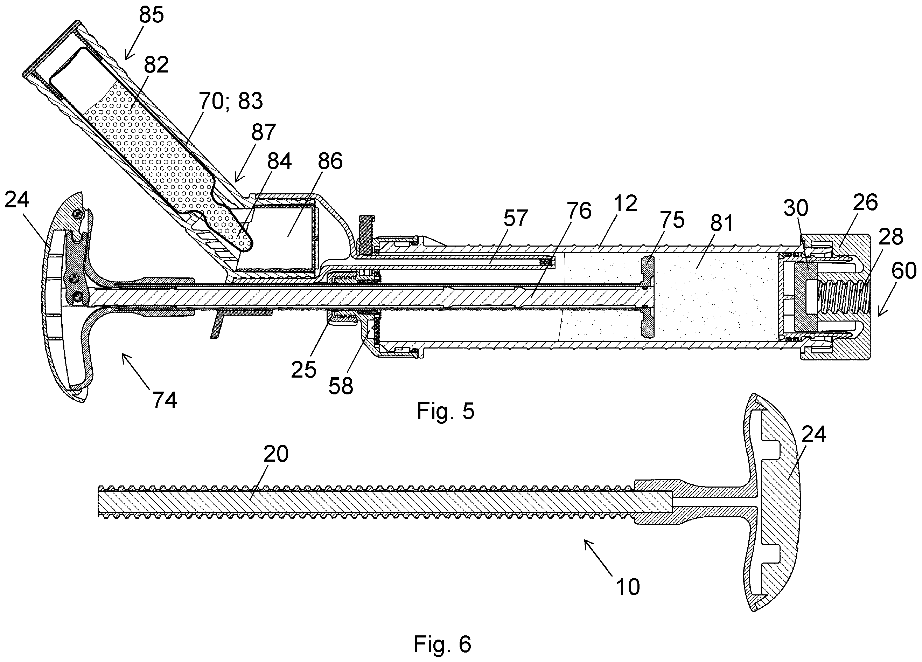

Figur 5 zeigt eine Ausführungsform des ersten Behälters 12, einer Vorrichtung 85 zum Lagern und Öffnen des zweiten Behälters 70 und einer Auspressvorrichtung 10, beispielsweise gemäß Figur 3 wie oben beschrieben. Die Vorrichtung 85 enthält den zweiten Behälter 70, der als Ampulle 83 mit einer zweiten Komponente 82, nämlich einer Monomerflüssigkeit ausgestaltet ist. Die erste Komponente 81 ist ein Knochenzementpulver, das sich im ersten Behälter befindet. Die Vorrichtung 85 umfasst einen Knickbereich 87, an dem ein Knicken der Vorrichtung 85 möglich ist, um den Kopf 84 der Ampulle 83 abzubrechen. Der Kopf 84 und etwaige Glassplitter werden dann von einem Sieb zurückgehalten und die Flüssigkeit kann über das Sammelvolumen 86, ggf. ein Filterelement, das Rohrelement 57 und ggf. ein weiteres Filterelement in den zweiten Behälter strömen. Dies kann durch Schwerkraft und/oder durch ein Vakuum erfolgen. Zum Anlegen eines Vakuums umfasst der erste Behälter 12 einen Stutzen (hier nicht gezeigt; vergl. Fig. 15A), der in eine Vakuumkammer 58 mündet. Diese ist mittels eines Gitters oder Siebs mit dem Innenraum des ersten Behälters 12 verbunden. Wird ein Vakuum angelegt, wird die Flüssigkeit durch das Rohrelement 57 in den Innenraum gesaugt.

In der hier gezeigten Ausführungsform umfasst die Adaptereinheit 26 der Auspressvorrichtung eine Aufstellfläche 60. Damit kann die Adaptereinheit zum Halten des ersten Behälters 12 dienen. Beispielsweise kann das Öffnen der Ampulle 83 und das z. B. zumindest teilweise schwerkraftbedingte Überführen der Flüssigkeit in den ersten Behälter 12 im mittels der Aufstellfläche 60 aufgestellten Zustand des ersten Behälters 12 erfolgen. Die Adaptereinheit 26 umfasst ein Innengewinde 28 zum Einschrauben der in Figur 6 gezeigten Gewindestange 20 der Auspressvorrichtung 10.

An der gegenüberliegenden Seite des ersten Behälters 12 ist eine Adaptereinheit 25 der Mischvorrichtung 74 angeordnet. Die Mischvorrichtung 74 umfasst ferner einen durch die Adaptereinheit 25 hindurchgeführten Stab 76, der ein Mischelement 75 mit einem Handgriff 24 verbindet.

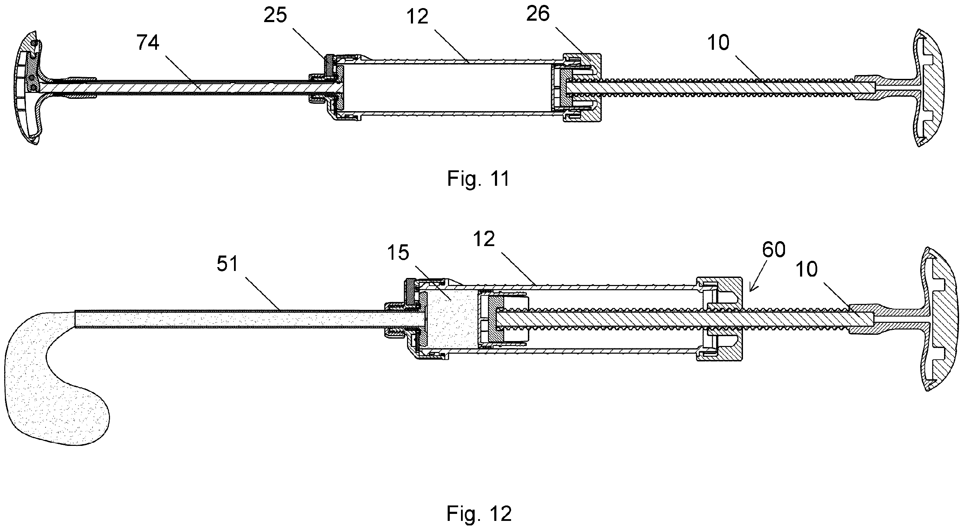

Figur 5 zeigt zudem einen Lösemechanismus der Mischvorrichtung 74. Wird ein Betätigungselement am Handgriff 24 auf geeignete Weise betätigt, insbesondere im vollständig herausgezogenen Zustand, kann der Handgriff 24 vom Stab 76 gelöst werden. Der Stab 76 ist hierbei als Hohlstab ausgeführt. Nach dem Lösen des Handgriffs kann der Stab 76 als Austragsrohr zum Austragen des Knochenzements benutzt werden. Das Mischelement 75 verbleibt im Inneren des ersten Behälters 12, beispielsweise im Bereich der links dargestellten Stirnseite und insbesondere in einem fixierten Zustand. Dieser Zustand ist auch in Figur 12 dargestellt.

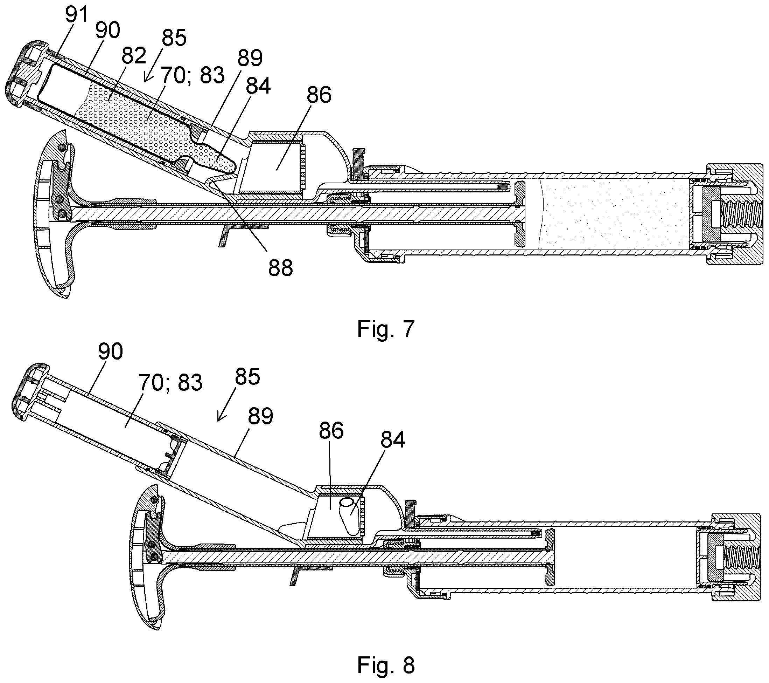

Eine alternative Ausgestaltung der Vorrichtung 85 zum Lagern und Öffnen des zweiten Behälters 70 ist in den Figuren 7 und 8 dargestellt. Die Vorrichtung 85 umfasst eine Innenhülle 90, in der die Ampulle 83 angeordnet ist, und eine Außenhülle 89, die die Innenhülle 90 zumindest teilweise umgibt. Die Innenhülle 90 ist axial in Bezug zur Außenhülle 89 zumindest in Richtung des ersten Behälters verschiebbar, beispielsweise nach Art eines Teleskops, kann jedoch von einer Sicherung 91, beispielsweise in Form eines abziehbaren Sicherungsclips, gehalten werden. Ist die Sicherung 91 eingesetzt, ist die Bewegung der Innenhülle 90 blockiert. Wird die Sicherung entfernt, kann die Innenhülle 90 in Richtung des ersten Behälters bewegt werden. Die Vorrichtung 85 umfasst ferner ein Scherelement 88 zum Abscheren des Kopfes 84 der Ampulle 73. Das Scherelement enthält typischerweise eine schräg zur Längserstreckung der Ampulle 83 ausgerichtete Oberfläche. Wird die Innenhülle 90 mit der Ampulle 83 in Richtung des ersten Behälters 12 geschoben, kontaktiert der Kopf 84 das Scherelement 88 und wird dadurch abgeschert.

In Figur 8 ist dargestellt, wie in der oben beschriebenen oder einer ähnlichen Ausführungsform, bei der die Vorrichtung 85 eine Außenhülle 89 und eine gegenüber der Außenhülle 89 bewegliche Innenhülle 90 aufweist, zum Pumpen der Flüssigkeit in den ersten Behälter 12 verwendet werden kann. Durch Herausziehen der Innenhülle 90, wie in Figur 8 gezeigt, wird das Volumen in der Vorrichtung 85 vergrößert und Luft strömt in die Vorrichtung 85 ein, beispielsweise durch ein geeignetes Ventil in einem Deckel der Vorrichtung 85. Beim anschließenden Hereinschieben verdrängt die Luft die in der Vorrichtung 85, beispielsweise im Sammelvolumen 86 befindliche Flüssigkeit, die zuvor aus der Ampulle 83 herausgeflossen ist, und drückt sie in den ersten Behälter. Auf diese Weise kann auf eine Vakuumquelle verzichtet werden und der Kit kann ohne jegliche zusätzliche Vorrichtung zum Zementieren verwendet werden.

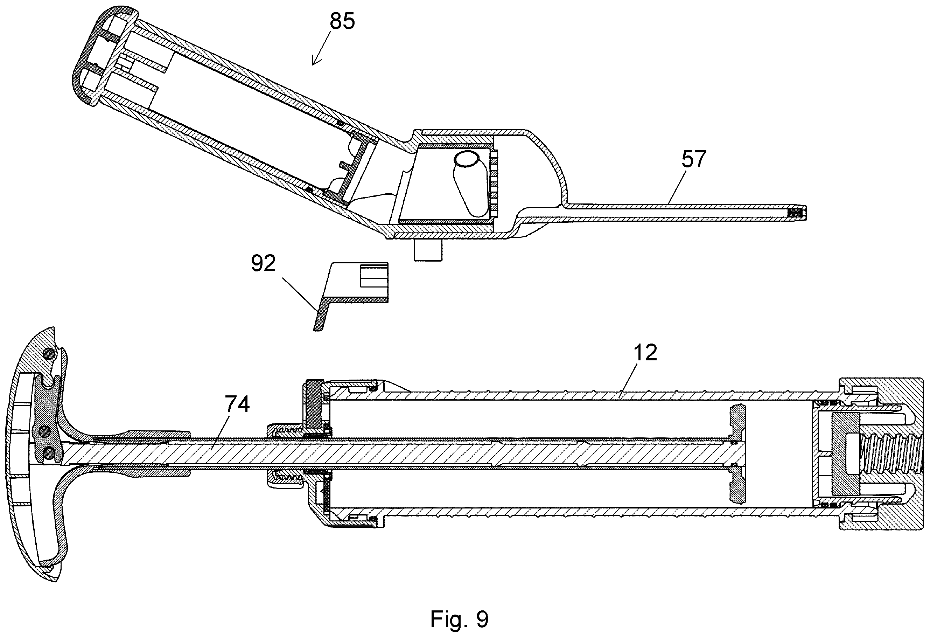

Figur 9 zeigt die Trennung der Vorrichtung 85 von dem ersten Behälter 12. Dabei kann zumindest ein Halteteil 92, das zuvor eine Verbindung hergestellt hat, entfernt werden. Anschließend ist ein leichteres Mischen und/oder Auspressen des Knochenzements möglich. Die Trennung kann irreversibel sein. Ein Verschlusselement, beispielsweise ein Schieber, kann die Öffnung im ersten Behälter 12 verschließen, durch die zuvor das Rohrelement 57 ragte.

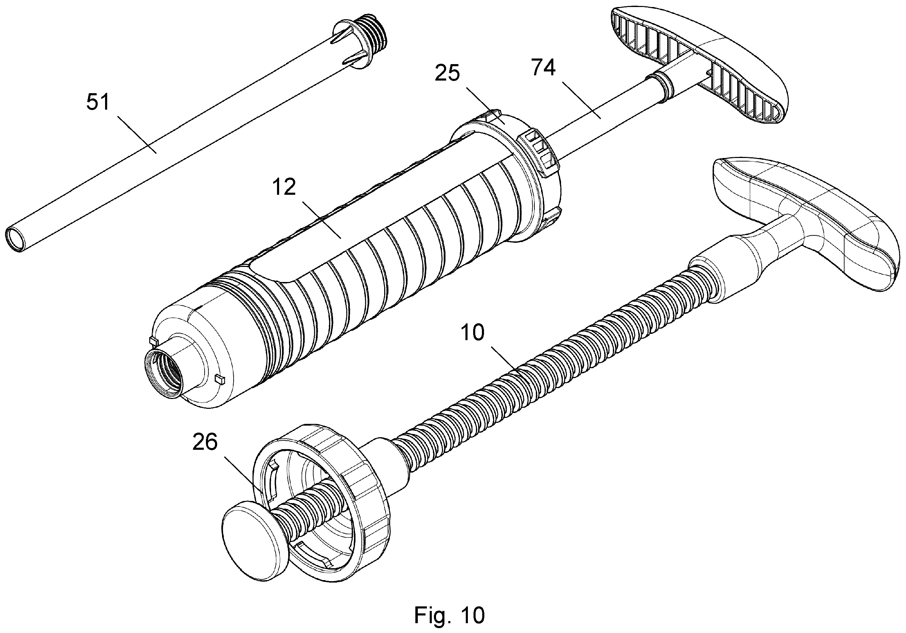

Figur 10 zeigt eine Ausführungsform, in der die Mischvorrichtung 74 mit dem ersten Behälter verbunden ist und separat davon die Auspressvorrichtung 10 und insbesondere ein Austragsrohr 51 vorhanden sind. Die Mischvorrichtung 74 umfasst eine Adaptereinheit 25 und die Auspressvorrichtung 10 umfasst eine Adaptereinheit 26. Beide Adaptereinheiten können auf derselben Seite des ersten Behälters 12 befestigt werden. Das Austragsrohr 51 kann auf der anderen Seite des ersten Behälters 12 befestigt werden.

Figur 11 zeigt eine andere Ausgestaltung, in der die Adaptereinheit 25 für die Mischvorrichtung 74 und die Adaptereinheit 26 für die Austragsvorrichtung an gegenüberliegenden Seiten der Vorrichtung angeordnet werden. Figur 12 zeigt das Auspressen des Knochenzements 15 mittels der Auspressvorrichtung 10 durch das Austragsrohr 51.

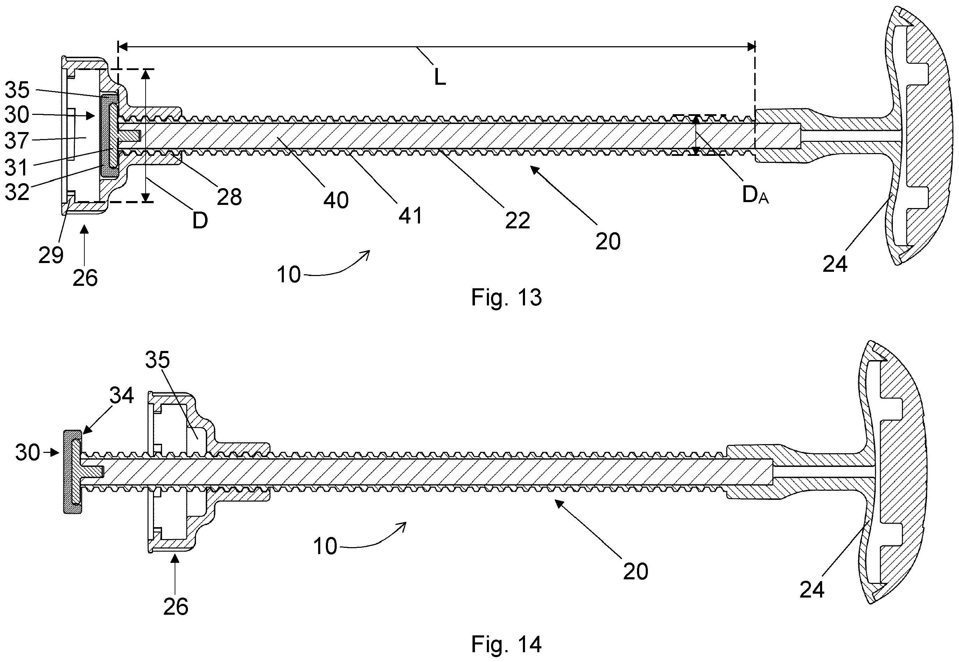

Die Figuren 13 und 14 zeigen eine Schnittzeichnung einer Auspressvorrichtung 10 entlang der Mittellängsachse. Die Auspressvorrichtung 10 umfasst eine Adaptereinheit 26 mit einem Verbindungsbereich 37 zum mechanischen Verbinden mit einem ersten Behälter. Der Verbindungsbereich 37 kann in der hier gezeigten Ausführungsform auf einen entsprechenden Bereich eines ersten Behälters aufgesteckt bzw. aufgedreht werden. In der hier gezeigten Ausführungsform hat die Adaptereinheit 26 Verbindungselemente 29 zur Herstellung einer Bajonettverbindung. Die Drehrichtung zum Befestigen der Bajonettverbindung entspricht dabei der Drehrichtung zum Einschrauben der Gewindestange 20.

Die Auspressvorrichtung 10 umfasst ferner eine Gewindestange 20 mit einem Außengewinde 22. Die Gewindestange 20 ist durch die Durchgangsöffnung 27 hindurchgeführt, sodass das Außengewinde 22 mit dem in der Durchgangsöffnung 27 befindlichen Innengewinde 28 im Eingriff ist. An der rechts dargestellten Seite der Gewindestange 20 befindet sich der manuell bedienbare Handgriff 24 zum Drehen. Wird die Gewindestange 20 gedreht, erfolgt eine axiale Bewegung der Gewindestange durch die Adaptereinheit 26, sodass der Knochenzement herausgepresst werden kann.

An der dem Handgriff 24 gegenüberliegenden Seite der Gewindestange 20 ist die Gewindestange 20 zum Ausüben eines Drucks bzw. einer Kraft auf den Knochenzement eingerichtet. Dazu kann ein Druckelement 30 vorhanden sein. Das Druckelement 30 dient der direkten oder indirekten Ausübung eines Drucks auf Knochenzement, um diesen aus einem ersten Behälter herauszupressen. Beispielsweise kann das Druckelement 30 einen Kolben des ersten Behälters bewegen, welcher wiederum den Knochenzement aus dem ersten Behälter herauspresst. Auf das Druckelement 30 wird weiter unten im Detail eingegangen.

Die Gewindestange 20 kann einen innenliegenden Kern, im hier gezeigten Beispiel einen Metallstab 40, und/oder eine Ummantelung, im hier gezeigten Beispiel eine Kunststoffummantelung 41, aufweisen. Der Metallstab 40 verläuft zentral im Inneren der Gewindestange 20 und trägt maßgeblich zur mechanischen Stabilität der Gewindestange 20 bei. Die Kunststoffummantelung 41 bildet das Außengewinde 22 aus und schützt den Metallstab 40 vor äußeren Einflüssen. Der Metallstab 40 ist bevorzugt nicht-rotationssymmetrisch ausgebildet, um eine drehfeste Verbindung zum Handgriff 24 und/oder zur Ummantelung zu gewährleisten.

Beispielsweise kann der Metallstab 40 sechseckig oder quadratisch sein. Insbesondere wird ein Metallstab 40 mit oder aus einem Edelstahl wie beispielsweise 316L verwendet. Insbesondere ist der Metallstab 40 vollständig von Kunststoff umhüllt. Insbesondere besteht das Gewinde aus Kunststoff. Auf diese Weise wird ein besonders leichtgängiges Gewinde bereitgestellt. Der Metallstab 40 wird vor äußeren Einflüssen geschützt. In einer Ausführungsform weist der Metallstab 40 einen Durchmesser von wenigstens 7 mm und/oder höchstens 10 mm, bevorzugt etwa oder genau 8 mm auf.

In einer Ausführungsform weist die Gewindestange 20 eine Länge L von wenigstens 15 cm und/oder höchstens 23 cm auf. Die Länge der Gewindestange 20 beträgt insbesondere wenigstens 17 cm, bevorzugt wenigstens 19 cm und/oder höchstens 23 cm, bevorzugt höchstens 21 cm. Die Länge L wird zwischen den äußersten Bereichen gemessen, in denen das Außengewinde 22 vorliegt. Zudem ist der Außendurchmesser D_{A} der Gewindestange 20 dargestellt, der in radialer Richtung zwischen den Erhebungen des Außengewindes 22 gemessen wird. Der Außendurchmesser D_{A} beträgt im hier gezeigten Beispiel zwischen 13,5 mm und 15 mm.

In einer Ausführungsform weist die Adaptereinheit einen Verbindungsbereich zum Aufstecken auf den ersten Behälter auf. In einer Ausführungsform weist der Verbindungsbereich einen Innendurchmesser D von wenigstens 3,3 cm und/oder höchstens 4,0 cm auf. In einer Ausgestaltung enthält die Gewindestange außer dem Metallstab keine Metallteile.

In Figur 13 ist eine vollständig herausgeschraubte Position der Gewindestange 20 aus der Adaptereinheit 26 dargestellt. Dagegen ist in Figur 14 eine Position gezeigt, in der die Gewindestange 20 bereits teilweise durch die Adaptereinheit 26 hindurchgedreht ist, wie dies beim Herauspressen von Knochenzement erfolgt.

In einer Ausführungsform weist die Adaptereinheit 26 eine Ausnehmung 35 auf, in welche das Druckelement 30 vollständig aufgenommen werden kann. Auf diese Weise verbleibt der auf der linken Seite dargestellte Verbindungsbereich 37 der Adaptereinheit 26 frei von dem Druckelement 30. So kann der Verbindungsbereich 37 über das zugehörige Ende des ersten Behälters gestülpt bzw. auf dieses aufgesteckt oder aufgeschraubt werden, ohne dass dabei das Druckelement 30 stört.

Das Druckelement 30 kann - wie in den Figuren 13 und 14 dargestellt - zweiteilig aufgebaut sein. Dabei kann ein erster Teil 31 fest mit der Gewindestange 20 verbunden sein, so dass er sich bei einer Drehung der Gewindestange 20 gemeinsam mit dieser dreht. Ein zweiter Teil 32 kann drehbar mit dem ersten Teil 31 verbunden sein, so dass der zweite Teil 32 sich beim Drehen der Gewindestange 20 nicht mit dreht. Auf diese Weise wird keine Drehbewegung, sondern lediglich eine axiale Druckkraft auf den Knochenzement 15 bzw. den Kolben ausgeübt. Die einander kontaktierenden Oberflächen des ersten Teils 31 des zweiten Teils 32 sind bevorzugt eben und glatt ausgeführt, um eine leichtgängige Drehung zu ermöglichen.

Im hier gezeigten Beispiel umgreift der zweite Teil 32 des Druckelements 30 den ersten Teil 31 des Druckelements 30 bzw. bildet mit diesem eine Hinterschneidung 34 aus. Auf der rechten Seite erstreckt sich die insbesondere umlaufende, äußere Krempe des zweiten Teils 32 nach innen und hintergreift den radial äußeren Rand des ersten Teils 31. Der erste Teil 31 und/oder der zweite Teil 32 kann in Form eines Tellers ausgeführt sein. Der zweite Teil 32 kann einen Durchmesser von wenigstens 20 mm und/oder höchstens 30 mm, beispielsweise ungefähr oder genau 25 mm aufweisen.

Unterschiedliche Ausgestaltungen des Druckelements 30 sind in den Figuren 15A bis 15C dargestellt. Grundsätzlich drückt das Druckelement 30 auf den Knochenzement, um diesen herauszupressen. Insbesondere erfolgt dies indirekt, wobei das Druckelement 30 auf einen Kolben 13 drückt, der wiederum mit dem Knochenzement in Kontakt steht. In Figur 15A umfasst das Druckelement 30 lediglich ein erstes Teil 31, das fest oder drehbar mit der Gewindestange 20 verbunden ist. Der Aufbau ist besonders einfach. Allerdings kann es zu einem Mitdrehen des Kolbens 13 kommen, was die Reibung und damit die zum Auspressen benötigte Kraft erhöht. In Figur 15B ist ein zweiteiliger Aufbau des Druckelements 30 dargestellt. Zwischen dem ersten Teil 31 und dem Kolben 13 ist ein zweites Teil 32 des Druckelements 32 vorhanden, welches drehbar in Bezug zum ersten Teil 31 angeordnet ist. Das zweite Teil 32 erstreckt sich über nahezu den gesamten Innendurchmesser des entsprechenden Bereichs des Kolbens, wobei vorteilhaft ein zumindest geringfügiger Abstand zwischen der inneren Umfangsfläche des Kolbens 13 und dem zweiten Teil 32 verbleiben sollte, damit hier keine Reibung auftritt. Das zweite Teil 32 kann eine flache Scheibe sein. Insbesondere befindet sich zwischen den beiden Teilen 31, 32 ein Schmiermitte, wie z. B. Silikon, um ein leichtgängiges Drehen zu ermöglichen. Das ersten Teil 31 kann drehfest mit der Gewindestange 20 verbunden sein. Es erfolgt eine Drehung zwischen dem ersten Teil 31 und dem zweiten Teil 32, aber keine Drehung zwischen dem zweiten Teil 32 und dem Kolben 13. Dies verringert die Reibung und damit die benötigte Kraft. Figur 15C zeigt eine weitere Ausführungsform, in der das erste Teil 31 als flache Scheibe ausgestaltet ist, die sich in einer Vertiefung des zweiten Teils 32 befindet. Die Gewindestange dreht hierbei lediglich das flache erste Teil 31 und das zweite Teil 32 dreht sich nicht mit dem Kolben 13 mit. Die Teile 31, 32 übertragen die Kraft der Auspressvorrichtung gleichmäßig und verhindern ein Verkippen des Kolbens 13.

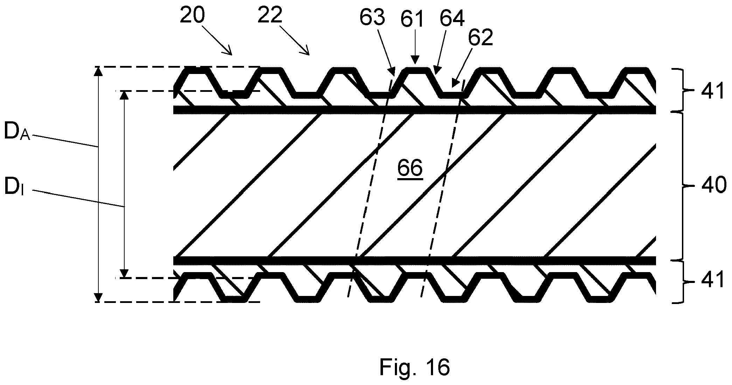

Figur 16 zeigt einen vergrößerten Ausschnitt eines Schnitts durch eine Gewindestange 20. Diese umfasst einen innenliegenden Metallstab 40 und eine Kunststoffummantelung 41, die den Metallstab 40 vollständig umschließt. Es ist der Außendurchmesser D_{A} der Gewindestange 20 dargestellt, der dem maximalen Durchmesser zwischen den beidseitigen Erhebungen des Außengewindes 22 entspricht. Zudem ist der Innendurchmesser D_{I} der Gewindestange 20 dargestellt, der dem minimalen Durchmesser zwischen den beidseitigen Vertiefungen oder Tälern des Außengewindes 22 entspricht. Das Gewinde hat eine Gewindetiefe zwischen 1 mm und 2 mm. Die Differenz zwischen Innendurchmesser D_{I} und Außendurchmesser D_{A} beträgt also zwischen 2 mm und 4 mm.

Ein Gewindegang 66 ist exemplarisch durch gestrichelte Linien abgegrenzt. Der Gewindegang entspricht einer vollständigen Drehung des Außengewindes 22 in Bezug zu dem korrespondierenden Innengewinde. Die Gewindestange 20 kann so eingerichtet sein, dass eine äußere Oberfläche eines Gewindegangs 66 des Außengewindes 22 höchstens 370 mm² beträgt. Die äußere Oberfläche des Gewindes bestimmt sich im hier gezeigten Beispiel eines Trapezgewindes als Summe aus der oberen Fläche 61, die der Fläche im Bereich der Erhebung des Gewindes entspricht, der unteren Fläche 62, die der Fläche im Bereich der Vertiefung des Gewindes entspricht, der ersten (aufsteigenden) Flankenfläche 63 sowie der zweiten (absteigenden) Flankenfläche 64.

In einer Ausgestaltung beträgt eine äußere Oberfläche eines Gewindegangs des Außengewindes höchstens 450 mm², höchstens 430 mm², höchstens 400 mm², höchstens 370 mm², höchstens 350 mm², höchstens 330 mm² oder höchstens 300 mm². In einer Ausgestaltung beträgt eine äußere Oberfläche eines Gewindegangs des Außengewindes mindestens 100 mm², mindestens 130 mm², mindestens 160 mm², mindestens 180 mm², mindestens 200 mm², mindestens 215 mm², mindestens 250 mm², mindestens 280 mm², mindestens 320 mm² oder mindestens 350 mm². Diese Werte können je nach Ausgestaltung und Materialien besonders geeignet sein.

In einem Beispiel hat die Gewindestange einen Außendurchmesser D_{A} von 14 mm, eine Gewindetiefe (Zahnhöhe) von 1,5 mm und eine Steigung von 4,5 mm. Diese Werte stellt einen guten Kompromiss zwischen Vortrieb pro Drehung und der notwendigen Kraft zum Drehen dar. Zudem besitzt diese Gewindestange 20 eine geeignete Selbsthemmung, hat also beim Auspressen keine Bestrebung, sich selbst zurückzudrehen.

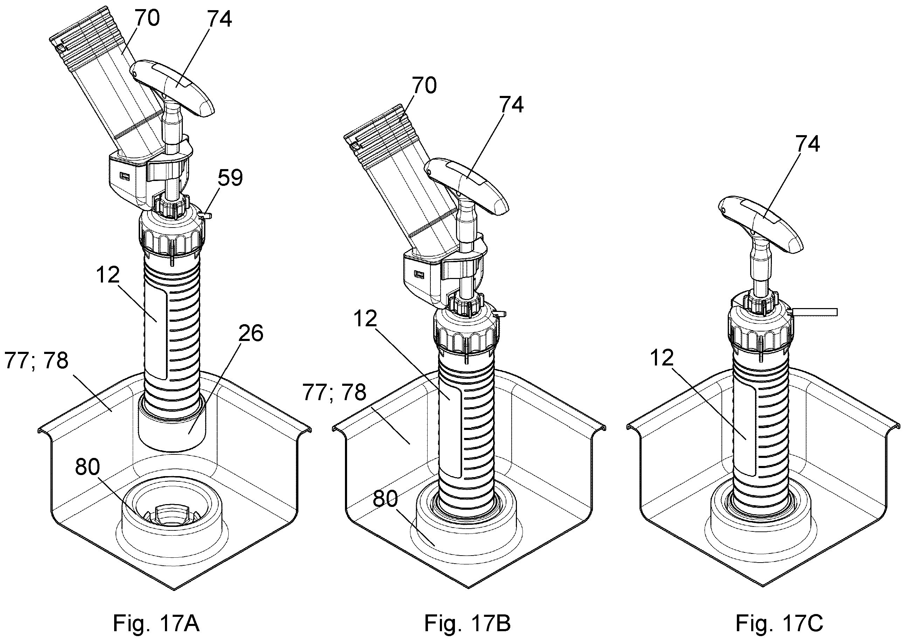

Die Figuren 17A bis 17B zeigen die Verwendung einer Halterung 80 im Packmittel 77. Mit dem ersten Behälter 12 ist an dessen der Vorrichtung 85 abgewandten Seite eine Adaptereinheit 26 insbesondere einer Auspressvorrichtung 10 verbunden. Die Adaptereinheit 26 umfasst eine Aufstellfläche 60. Die Aufstellfläche 60 korrespondiert mit der Halterung 80, die an der Innenseite des Packmittels 77 angeordnet oder ausgebildet ist, beispielsweise durch Tiefziehen. Sowohl die Aufstellfläche 60 als auch die Halterung 80 sind als dreidimensionale Strukturen ausgebildet, die eine gute Verzahnung und somit einen stabilen Halt gewährleisten. Ist die Adaptereinheit 26 dagegen als Hohlzylinder ausgebildet, ist ein Aufstellen auf einer flachen Oberfläche möglich.

Figur 17A zeigt den Zustand kurz vor dem Aufstellen des ersten Behälters 12 auf die Aufstellfläche 80. Figur 17B zeigt den aufgestellten Zustand. Die Vorrichtung zum Lagern und Öffnen des zweiten Behälters mit dem zweiten Behälter 70 sowie die Mischvorrichtung sind mit dem ersten Behälter verbunden. In Figur 17C sind die genannten Vorrichtungen entfernt. Nun kann das Mischen erfolgen. Durch die feste und sichere Aufstellung des ersten Behälters 12 kann dies besonders einfach und sicher erfolgen.

**Bezugszeichenliste**

| | |
|---|---|
| Kit | 1 |
| Auspressvorrichtung | 10 |
| Pumpeinrichtung | 11 |
| Erster Behälter | 12 |
| Kolben | 13 |
| Knochenzement | 15 |
| Gewindestange | 20 |
| Außengewinde | 22 |
| Handgriff | 24 |
| Adaptereinheit | 25 |
| Adaptereinheit | 26 |
| Durchgangsöffnung | 27 |
| Innengewinde | 28 |
| Verbindungselement | 29 |
| Druckelement | 30 |
| Erster Teil | 31 |
| Zweiter Teil | 32 |
| Hinterschneidung | 34 |
| Ausnehmung | 35 |
| Verbindungsbereich | 37 |
| Metallstab | 40 |
| Kunststoffummantelung | 41 |
| Vakuumschlauch | 50 |
| Austragsrohr | 51 |
| Kniepressurizer | 52 |
| Hüftpressurizer | 53 |
| Knieschnorchel | 54 |
| Flachschnorchel | 55 |
| Rohrelement | 57 |
| Vakuumkammer | 58 |
| Stutzen | 59 |
| Aufstellfläche | 60 |
| Obere Fläche | 61 |
| Untere Fläche | 62 |
| Erste Flankenfläche | 63 |
| Zweite Flankenfläche | 64 |
| Gewindegang | 66 |
| Zweiter Behälter | 70 |
| Mischvorrichtung | 74 |
| Mischelement | 75 |
| Stab | 76 |
| Packmittel | 77 |
| Schale | 78 |
| Deckel | 79 |
| Halterung | 80 |
| Erste Komponente | 81 |
| Zweite Komponente | 82 |
| Ampulle | 83 |
| Kopf | 84 |
| Vorrichtung | 85 |
| Sammelvolumen | 86 |
| Knickbereich | 87 |
| Scherelement | 88 |
| Außenhülle | 89 |
| Innenhülle | 90 |
| Sicherung | 91 |
| Halteteil | 92 |
| Innendurchmesser | D |
| Innendurchmesser | D_{I} |
| Außendurchmesser | D_{A} |
| Länge | L |
| Axiale Erstreckung | A |
| Axiale Erstreckung | B1 |

## Patentansprüche

1. Kit (1) zum Herstellen und Auspressen von Knochenzement (15), umfassend
- Einen ersten Behälter (12), der eine erste Komponente (81) zur Herstellung von Knochenzement (15) enthält,
- Einen zweiten Behälter (70), der eine zweite Komponente (82) zur Herstellung von Knochenzement (15) enthält,
- Eine Mischvorrichtung (74) zum manuellen Mischen der ersten Komponente (81) und der zweiten Komponente (82) im ersten Behälter (12) zwecks Herstellung von Knochenzement (15),
- Eine Auspressvorrichtung (10) zum manuellen Auspressen des hergestellten Knochenzements (15) aus dem ersten Behälter (12).

2. Kit (1) nach dem vorhergehenden Anspruch, **dadurch gekennzeichnet, dass** die Mischvorrichtung (74) zumindest teilweise im ersten Behälter (12) angeordnet ist, wobei die Auspressvorrichtung (10) eine axiale Erstreckung A aufweist und der erste Behälter (12) mit der Mischvorrichtung (74) eine axiale Erstreckung B aufweist, wobei für das Verhältnis A / B gilt:
A/ B ≥ 0,9 und/oderA/ B ≤ 1,5.

3. Kit (1) nach einem der vorhergehenden Ansprüche, **dadurch gekennzeichnet, dass** die Auspressvorrichtung (10) eine Gewindestange (20) mit einem Außengewinde (22), einen Handgriff (24) zum Drehen der Gewindestange (20) sowie eine Adaptereinheit (26) zum mechanischen Verbinden mit dem ersten Behälter (12) aufweist, wobei die Adaptereinheit (26) eine Durchgangsöffnung (27) mit einem Innengewinde (28) für die Gewindestange (20) aufweist.

4. Kit (1) nach dem vorhergehenden Anspruch, **dadurch gekennzeichnet, dass** die äußere Oberfläche eines Gewindegangs (66) des Außengewindes (22) höchstens 370 mm² und/oder wenigstens 230 mm² beträgt.

5. Kit (1) nach einem der vorhergehenden Ansprüche, **dadurch gekennzeichnet, dass** die Auspressvorrichtung (10) dazu eingerichtet ist, den Knochenzement (15) mit einer Auspresskraft von mindestens 0,7 kN, insbesondere mindestens 1,0 kN, aus dem ersten Behälter (12) zu pressen.

6. Kit (1) nach einem der vorhergehenden Ansprüche, **dadurch gekennzeichnet, dass** der Kit (1) ein Packmittel (77) umfasst und die Bestandteile des Kits (1) insbesondere luftdicht und/oder steril von dem Packmittel (77) eingeschlossen sind.

7. Kit (1) nach einem der vorhergehenden Ansprüche, **dadurch gekennzeichnet, dass** der erste Behälter (12) als erste Komponente (81) eine Menge von mindestens 40 g eines PMMA-Knochenzementpulvers enthält und/oder dass der zweite Behälter (70) als zweite Komponente (82) eine Menge von mindestens 18 ml einer Monomerflüssigkeit enthält.

8. Kit (1) nach einem der vorhergehenden Ansprüche, **dadurch gekennzeichnet, dass** der zweite Behälter (70) in einer Vorrichtung (85) zum Lagern und Öffnen des zweiten Behälters (70) angeordnet ist, wobei die Vorrichtung (85) zum Lagern und Öffnen des zweiten Behälters (70) fluidtechnisch mit dem ersten Behälter (12) verbunden oder verbindbar ist, um die zweite Komponente (82) in den ersten Behälter (12) zu überführen.

9. Kit (1) nach einem der vorhergehenden Ansprüche, **dadurch gekennzeichnet, dass** der erste Behälter (12) und/oder der zweite Behälter (70) separat von der Auspressvorrichtung (10) angeordnet ist.

10. Kit (1) nach einem der vorhergehenden Ansprüche, **dadurch gekennzeichnet, dass** die Mischvorrichtung (74) zumindest teilweise im ersten Behälter (12) angeordnet ist, wobei der erste Behälter (12) mit der Mischvorrichtung (74) und die Auspressvorrichtung (10) parallel angeordnet sind, und zwar bevorzugt übereinander.

11. Kit (1) nach einem der vorhergehenden Ansprüche, **dadurch gekennzeichnet, dass** der erste Behälter (12), der zweite Behälter (70), die Mischvorrichtung (74) und die Auspressvorrichtung (10) insgesamt zu maximal 20% aus Metall bestehen.

12. Kit (1) nach einem der neun vorhergehenden Ansprüche, **dadurch gekennzeichnet, dass** die Adaptereinheit (26) mit dem ersten Behälter (12) mechanisch verbunden oder verbindbar ist, wobei die Adaptereinheit (26) eine Aufstellfläche (60) zum Aufstellen der Adaptereinheit (26) mit dem ersten Behälter (12) auf eine glatte Oberfläche und/oder auf eine Halterung (80) des Packmittels (77) aufweist,
insbesondere wobei das Packmittel (77) eine entsprechende Halterung (80) aufweist.

13. Kit (1) nach einem der fünf vorhergehenden Ansprüche, **dadurch gekennzeichnet, dass** die Vorrichtung (85) zum Lagern und Öffnen des zweiten Behälters (70)
- einen Knickbereich (87) zum Knicken der Vorrichtung (85) aufweist, sodass der zweite Behälter (70) durch Knicken geöffnet werden kann, oder
- eine Außenhülle (89), eine Innenhülle (90) und ein Scherelement (88) aufweist, wobei die Außenhülle (89) und die Innenhülle (90) relativ zu einander verschieblich sind und der zweite Behälter (70) durch das relative Verschieben in Bezug zum Scherelement (88) bewegt wird, um den zweiten Behälter (70) durch Abscheren eines Teils des zweiten Behälters (70) zu öffnen.

14. Verwendung eines Kits (1) nach einem der vorhergehenden Ansprüche zum Herauspressen einer Menge zwischen 50 g und 130 g Knochenzement (15) aus dem ersten Behälter (12).

15. Verfahren zur Verwendung eines Kits (1) nach einem der vorhergehenden Ansprüche, umfassend
- Mischen der ersten Komponente (81) mit der zweiten Komponente (82) im ersten Behälter (12) unter Verwendung der Mischvorrichtung (74) zur Herstellung von Knochenzement (15),
- Entnahme der Auspressvorrichtung (10) aus einem Packmittel (77) des Kits (1),
- Auspressen des hergestellten Knochenzements (15) aus dem ersten Behälter (12) unter Verwendung der Auspressvorrichtung (10).
